(19) Europäisches Patentamt
European Patent Office
Office européen des brevets

(11) EP 1 706 140 B1

(12) **EUROPEAN PATENT SPECIFICATION**

(45) Date of publication and mention of the grant of the patent:
**15.06.2011 Bulletin 2011/24**

(21) Application number: **05722399.2**

(22) Date of filing: **07.01.2005**

(51) Int Cl.:
***A61K 39/00*** *(2006.01)* ***A61K 39/29*** *(2006.01)*
***A61K 48/00*** *(2006.01)*

(86) International application number:
**PCT/US2005/000710**

(87) International publication number:
**WO 2005/067966 (28.07.2005 Gazette 2005/30)**

(54) **METHODS FOR TAILORING THE IMMUNE RESPONSE TO AN ANTIGEN OR IMMUNOGEN**

VERFAHREN ZUR ANPASSUNG DER IMMUNANTWORT AN EIN ANTIGEN ODER IMMUNOGEN

METHODES D'ADAPTATION DE LA REPONSE IMMUNITAIRE A UN ANTIGENE OU A UN IMMUNOGENE

(84) Designated Contracting States:
**AT BE BG CH CY CZ DE DK EE ES FI FR GB GR HU IE IS IT LI LT LU MC NL PL PT RO SE SI SK TR**

(30) Priority: **07.01.2004 US 534923 P**

(43) Date of publication of application:
**04.10.2006 Bulletin 2006/40**

(73) Proprietors:
- **Biomedical Research Models, Inc.**
  **Worcester, MA 01606 (US)**
- **Oral Vaccine Technologies, Inc.**
  **Las Vegas, 89121 (US)**

(72) Inventors:
- **YANG, Kejian**
  **Northborough, MA (US)**
- **WHALEN, Barbara, J.**
  **Shrewsbury, MA (US)**
- **KISLAUSKIS, Edward, H.**
  **Medway, MA 02053 (US)**
- **GUBERSKI, Dennis, L.**
  **Worcester, MA 01606 (US)**

(74) Representative: **Muir, Justine Claire**
**BioScience Patents Ltd**
**The Granary**
**St Mary's Lane**
**Ticehurst**
**East Sussex TN5 7AX (GB)**

(56) References cited:
**WO-A-00/11140**

- **DAVIS HEATHER L ET AL: "DNA-mediated immunization to hepatitis B surface antigen: Longevity of primary response and effect of boost" VACCINE, vol. 14, no. 9, 1996, pages 910-915, XP004069580 ISSN: 0264-410X**
- **RICHMOND J F L ET AL: "STUDIES OF THE NEUTRALIZING ACTIVITY AND AVIDITY OF ANTI-HUMAN IMMUNODEFICIENCY VIRUS TYPE 1 ENV ANTIBODY ELICITED BY DNA PRIMING AND PROTEIN BOOSTING" JOURNAL OF VIROLOGY, THE AMERICAN SOCIETY FOR MICROBIOLOGY, US, vol. 72, no. 11, November 1998 (1998-11), pages 9092-9100, XP002939091 ISSN: 0022-538X**
- **DONG-JI ZHANG ET AL: "Priming with Chlamydia trachomatis major outer membrane protein (MOMP) DNA followed by MOMP ISCOM boosting enhances protection and is associated with increased immunoglobulin A and Th1 cellular immune responses" INFECTION AND IMMUNITY, AMERICAN SOCIETY FOR MICROBIOLOGY. WASHINGTON, US, vol. 68, no. 6, June 2000 (2000-06), pages 3074-3078, XP002164567 ISSN: 0019-9567**
- **SIN J-I ET AL: "DNA PRIMING-PROTEIN BOOSTING ENHANCES BOTH ANTIGEN-SPECIFIC ANTIBODY AND TH1-TYPE CELLULAR IMMUNE RESPONSES IN A MURINE HERPES SIMPLEX VIRUS-2 GD VACCINE MODEL" DNA AND CELL BIOLOGY, NEW YORK, NY, US, vol. 18, no. 10, 1999, pages 771-779, XP009029373 ISSN: 1044-5498**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 706 140 B1

Remarks:

The file contains technical information submitted after the application was filed and not included in this specification

## Description

### Cross-Reference to Related Applications

The present application claims the benefit of U.S. Provisional Application No. 60/534,923, filed January 7, 2004.

### Governmental Funding

The invention described herein was supported, in whole or in part, by Grant R31/CCR922413-01; R31/CCR924378-01 from the Centers for Disease Control and Prevention. The United States government has certain rights in the invention.

### Background of the Invention

The development of effective preventative and therapeutic vaccines against a variety of agents (such as infectious microorganisms) is an important objective for disease control worldwide. In many cases, the most effective vaccine specifically targets the most common entry portal for microorganisms, the mucosal surfaces of the body, including nasal passages, lung, reproductive tracts and the gut.

Protective immunity against specific pathogens is achieved by humoral, cellular and mucosal immune responses. Humoral or antibody responses are important in pathogen neutralization and can be very effective in some infectious diseases. Antibodies circulate in the blood, and provide a measure of the overall humoral immune responses. Cell mediated immunity, specifically the involvement of cytotoxic T lymphocytes (CTL) is necessary for protective immunity against many intracellular pathogens and cancer. Localized immunity at the mucosal surfaces of the nasal passages, lung, reproductive tracts and gut are also crucial for effective immune responses against pathogens which access the body through these sites. The hallmark of effective mucosal immune responses is the local production of secretory IgA antibody at mucosal surfaces. IgA also circulates in the blood and its levels there provide a gauge on the overall mucosal immune responses. Mucosal IgG is also important in neutralizing certain pathogens. Repeated immunization may result in enhanced immune responses and confer increased protection. A priming immunization establishes a base level of immunity and can lead to the development of T and B cell memory. A booster immunization at a later time can mobilize these memory cells and lead to higher and more specific immune responses.

Most immune responses are regulated by T lymphocytes, which initiate and shape the nature of the response. As immune responses mature, $CD4^+$ T lymphocytes can become polarized towards T helper type 1 (Th1) or T helper type 2 (Th2) immune responses. The hallmark of Th1 and Th2-type responses is the predominant pattern of cytokines that are present. Th1 responses are characterized by high levels of IFN-$\gamma$ and low levels of IL-4 and IL-10, while Th2 responses are characterized by low levels of IFN-$\gamma$ and high levels of IL-4 and IL-10. These cytokines play an important role in determining the functional capabilities of the T cells. Th2-type responses lead to the preferred production of antibodies of the IgG1 subclass, with little or no generation of CTLs. Th1-type responses lead to the preferred production of antibodies of the IgG2a subclass and induction of CTLs that can effectively kill cells infected with viruses or other organisms.

Table A below summarizes the immunological characteristics of Th1 and Th2 polarized immune responses. Th1 polarized responses are typically generated during infections with viruses or bacteria. In contrast, Th2 polarized responses are often observed in parasitic infections, in allergic responses, and by conventional alum-based intramuscularly delivered protein vaccines that are used in humans. Genetics can also determine the type of immune responses generated. For example, Th1 responses predominate in the C57BL/6 strain of mouse, while Th2 responses predominate in the Balb/c strain of mouse. Immune responses may also consist of both Th1 and Th2 components, affording protection by both humoral and cell mediated arms of the immune response. Direct determination of the frequencies of cytokine producing cells is accomplished by the use of ELISPOT (Enzyme Linked Immunosorbent SPOT assays) or by immunofluorescence staining to reveal intracellular cytokine production. Serum IgG1: IgG2a ratios are also a widely accepted and followed criteria to determine T helper types (Table A). An IgG1 to IgG2a ratio for balanced Th1 and Th2 response would be between 0.5 and 2.0.

Table A. Characteristics of Th1 and Th2 polarized T cell responses.

| Immune responses | Th1-type immune responses | Th2-type immune responses |
|---|---|---|
| Humoral immunity | Serum IgG1/IgG2a < 0.5 | Serum IgG1/IgG2a > 2.0 |
| T cell cytokine secretion | ↑ IFN-$\gamma$, ↓ IL-10 and IL-4 | ↑ IL-10 and IL-4, ↓ IFN-$\gamma$ |
| CTL | High | Low or absent |

(continued)

| Immune responses | Th1-type immune responses | Th2-type immune responses |
|---|---|---|
| Prototypical mouse strains | C57BL/6 | Balb/c |

As used herein, "T helper type 1 response" and "Th1 response" are used interchangeably to refer to a range of host animal responses including one or more, usually all the characteristics listed in the middle column of Table A above. These characteristics include a ratio of IgG1:IgG2a of no greater than 0.5; increased IFN-γ (and other Th1 cytokines) secretion by T helper 1 cells and decreased IL-10 and IL-4 (and other Th2 cytokines) secretion by T helper 2 cells; and high CTL activity.

Similarly, as used herein, "T helper type 2 response" and "Th2 response" are used interchangeably to refer to a range of host animal responses including one or more, usually all the characteristics listed in the right column of Table A above. These characteristics include a ration of IgG1:IgG2a of no less than 2.0; decreased IFN-γ (and other Th1 cytokines) secretion by T helper 1 cells and increased IL-10 and IL-4 (and other Th2 cytokines) secretion by T helper 2 cells; and low or absent CTL activity.

Vaccine delivery takes a variety of forms, depending on the agent to be delivered and the administration route. Vaccine delivery systems are often designed to administer vaccines to specific areas of the body. In the gastrointestinal tract it is important that the vaccine not be degraded or eliminated before it has had a chance to exert a localized effect or to pass into the bloodstream or to interact with lymphoid tissue in the local environment. In the nasopharyngeal tract, it is important that the vaccine remains in proximity to the absorptive cells; it is also important that antigens remain in contact with lymphoid cells before the antigen is washed through the nasal tract or is swallowed. Further, it is important that antigen, vehicle, and immune stimulator are co-delivered in a single complex.

Traditionally, immunization has been accomplished by administering inactivated whole organisms or cells, extracts of microorganisms or cells, or isolated components of those such as protein or peptide antigens. Immunization has been achieved by oral, intranasal (IN), or intramuscular (IM) administration of attenuated live organisms. Typically, humans and animals are injected with such compositions in the presence of preservatives, adjuvants, and other excipients via intramuscular or subcutaneous routes to elicit protective immunity in normal pediatric or adult vaccines in common use. Parenteral immunization rarely is capable of eliciting an effective mucosal immune response that results in antibody production, particularly production of immunoglobulin A (IgA), which is important as a first defense barrier to invading microorganisms.

Currently, only live attenuated viruses or bacteria are capable of inducing protective immune response in humans or animals when administered orally and or intranasally. To date, very few vaccines have been developed that can be administered by the oral, intranasal or intravaginal route and these vaccines are invariably live organisms such as the oral polio virus vaccines and a commonly used oral salmonella typhoid fever vaccine. For example, live attenuated influenza virus prepared by cold-adaptation (FluMist™) can be used to vaccinate humans against influenza by intranasal administration. There have been concerns about the potential risks associated with use of live virus vectored or based vaccine due to the natural tendency of genetic mutation as well as the efficacy and safety issues when applied in immune compromised patients.

Typically, vaccines in common use are prepared from cellular extracts or produced by recombinant production methodology; these vaccines are usually composed of proteins, microbial toxoids, inactivated whole viruses, or polysaccharides of microbial origin. These types of materials are usually effective in induction of disease protective immune responses when administered by injection. However, such materials are poorly immunogenic or nonimmunogenic when administered through oral or intranasal administration, even though the intestinal and nasopharyngeal tract of humans and most animals is richly endowed with cells and tissues capable of induction of immune response to non-host antigens. One of the main failures of orally or intranasally administered vaccines is that antigens are poorly absorbed and unstable during passage. Proteins and peptides that are administered orally are invariably degraded in the GI tract by action of proteases and other hydrolytic enzymes, and stomach acid. Thus, vaccines composed of subcellular immunogens are ineffective when administered by the oral or intranasal routes. Therefore, compositions that can be used repeatedly as antigen protective vehicles as well as effective adjuvants that can induce effective immune responses after oral or intranasal administration would provide a more useful and convenient means to vaccinate animals and humans.

Mucosa-Associated Lymphoid Tissue (MALT) is a network system consisting of the gastrointestinal tract (gut-associated lymphoid tissue, GALT), mammary glands, reproductive tract and respiratory tract (bronchus- and nasal-associated lymphoid tissue, BALT and NALT). One of the unique features of the common mucosal immune system is that immunological induction at one mucosal site often results in immune responses at distal mucosal sites. This is due to lymphocyte migration via the high endothelial venules. Distinct differences have been observed however upon administration of vaccines to different mucosal surfaces and compartmentalization of responses can occur. Further, some mucosal sites induce better responses at distal sites and not all distal sites respond equally.

Intranasal immunization is an effective route for stimulating mucosal immunity to various pathogens, soluble proteins and microparticle-delivered antigens. Intranasal immunization has the added benefit of inducing both local and distal mucosal immune responses. For example, intranasal vaccination can elicit immunity in the genito-urinary tract where direct immunization is often hampered by epithelial cell turnover and hormonal influences. (Csencsits KL et al. 1999. JI. 1382-1389). Intranasal vaccination can induce strong antigen specific immune responses through NALT, or through deep lung immunization. NALT, the major mucosal inductive site for the upper respiratory tract, is important for the development of mucosal immunity locally and distally to intranasally immunized antigen. Mucosal IgA and IgG responses and elevated CTL responses to viral peptides have been observed after stimulation of NALT. The lung is an immune-responsive organ that can locally produce IgA, IgG, IgE and cell-mediated immune responses (Liu M et al. 1982. J Immunology. 129(6):2653-2661) (Bice DE, et al 1980. Int Arch Allergy Appl Immunol. 63(4):438-45). Protective immune responses in the lung have been demonstrated after influenza virus infection or vaccination with influenza vaccine while long-term antibody production after lung immunization and challenge have been related to lung associated tissues (Bice DE, et al 1993. Am J Respir Cell Mol Biol. 8(6):662-667).

Mucosa-Associated Lymphoid Tissue (MALT) is a network system consisting of the gastrointestinal tract (gut-associated lymphoid tissue, GALT), mammary glands, reproductive tract and respiratory tract (bronchus- and nasal-associated lymphoid tissue, BALT and NALT). One of the unique features of the common mucosal immune system is that immunological induction at one mucosal site often results in immune responses at distal mucosal sites. This is due to lymphocyte migration via the high endothelial venules. Distinct differences have been observed however upon administration of vaccines to different mucosal surfaces and compartmentalization of responses can occur. Further, some mucosal sites induce better responses at distal sites and not all distal sites respond equally.

Intranasal immunization is an effective route for stimulating mucosal immunity to various pathogens, soluble proteins and microparticle-delivered antigens. Intranasal immunization has the added benefit of inducing both local and distal mucosal immune responses. For example, intranasal vaccination can elicit immunity in the genito-urinary tract where direct immunization is often hampered by epithelial cell turnover and hormonal influences. (Csencsits KL et al. 1999. JI. 1382-1389). Intranasal vaccination can induce strong antigen specific immune responses through NALT, or through deep lung immunization. NALT, the major mucosal inductive site for the upper respiratory tract, is important for the development of mucosal immunity locally and distally to intranasally immunized antigen. Mucosal IgA and IgG responses and elevated CTL responses to viral peptides have been observed after stimulation of NALT. The lung is an immune-responsive organ that can locally produce IgA, IgG, IgE and cell-mediated immune responses (Liu M et al. 1982. J Immunology. 129(6):2653-2661) (Bice DE, et al 1980. Int Arch Allergy Appl Immunol. 63(4):438-45). Protective immune responses in the lung have been demonstrated after influenza virus infection or vaccination with influenza vaccine while long-term antibody production after lung immunization and challenge have been related to lung associated tissues (Bice DE, et al 1993. Am J Respir Cell Mol Biol. 8(6):662-667). Optimal upper respiratory tract immunity is induced by combined upper and lower respiratory tract inoculation (Thompson AH. Vaccine 17: 1404-15).

DNA immunization is an approach for generating protective immunity against infectious diseases (Liu et al., Ann. N.Y. Acad. Sci. 772, 1995). Unlike protein or peptide based subunit vaccines, DNA immunization provides protective immunity through expression of foreign proteins by host cells, thus allowing the presentation of antigen to the immune system in a manner more analogous to that which occurs during infection with viruses or intracellular pathogens (Pardoll and Beckerieg, Immunity 3: 165, 1995; McDonnell and Askari, N. Engl. J. Med. 334: 42, 1996). Although considerable interest has been generated by this technique, successful immunity has been most consistently induced by DNA immunization for viral diseases (Manickan et al., J. Immunol. 155: 259, 1995). Results have been more variable with non-viral pathogens which may reflect differences in the nature of the pathogens, in the immunizing antigens chosen, and in the routes of immunization (Sedegah et al., Proc. Natl. Acad. Sci. USA 91: 9866, 1994; Barry et al., Nature 377: 632, 1995; Xu and Liew, Vaccine 12: 1534, 1994). Further development of DNA vaccination will depend on elucidating the underlying immunological mechanisms and broadening its application to other infectious diseases for which existing strategies of vaccine development have failed.

DNA vaccines were well tolerated in humans according to several recent clinical trials, but there is an urgent need to achieve a better immune potency. Hence, a number of second generation DNA vaccines are in development using various systems and devices. In addition, a new approach to immunization, called mixed modality vaccination or prime-boost, is being evaluated. It involves an initial vaccination that uses one type of vaccine, followed by a boost using a different type of vaccine. For example, promising preclinical results have been obtained by immunizing first with DNA then boosting with a vaccinia or adenovirus vector encoding the same antigen, or with a recombinant protein of the same antigen that the DNA vaccine encoded. WO-A-00/11140 discloses a method for eliciting an immune response in a mammal by administering a priming composition comprising a DNA sequence encoding an antigen followed by intranasal administration of a boosting composition comprising DNA encoding the antigen. However, there are concerns about vaccinia and adenovirus to be used as a booster. Because preexisting antibody against the viral vector would block immune responses, these recombinant viruses usually can not be used repeatedly and there is also a safety concern especially for immune compromised patients. Furthermore, pure proteins used as a booster by parenteral immunization routes generally can not induce mucosal immune response or enhance cell-mediated immune response.

Protective mucosal immune responses are crucial in controlling diseases caused by many naturally occurring infectious organisms and microorganisms that may be used in bioterrorism. Cell mediated immune responses including CTLs are also important in controlling intracellular pathogens both systemically and at mucosal sites. Traditional intramuscularly delivered protein vaccines, which use alum as an adjuvant, generate a typical Th2 response with mainly IgG1 antibody responses, but fail to stimulate CTL responses or local immunity at mucosal sites. There is therefore a need for effective vaccines that are capable of eliciting a wide range of desired immune responses to protect the animal from disease.

## Summary of the invention

In one aspect the invention relates to a priming preparation for intramuscular administration comprising a nucleic acid encoding an antigen: and a boosting preparation for intranasal administration comprising the antigen encapsulated in liposomes, for eliciting an immune response in an animal wherein administration of the boosting preparation produces an immune response comprising:

(1) an increase in the serum level of antigen-specific IgA compared to the serum level of antigen-specific IgA prior to the administration of the boosting preparation and,
(2) an increase compared to an untreated animal in the proportion of antigen specific CD8+IFN$\gamma$+ cytotoxic T lymphocytes in both the lung and the spleen.

Preferred aspects of the composition are detailed in claims 2 to 10 and claims 18.

In another aspect the invention relates to a kit for eliciting an immune response in a host animal comprising:

a) a first immunising component for intramuscular administration comprising a nucleic acid encoding an antigen;
b) a second immunising component for intranasal administration comprising the antigen encapsulated in liposomes;
c) an instruction for a user to administer to the animal the first immunizing component followed by administering the second immunizing component to elicit an immune response in the animal Preferred aspects of the kit are detailed in claims 12 to 17 and 19.

A method for eliciting a desired immune response in an animal, comprising administering to the animal a priming preparation comprising a nucleic acid sequence encoding an antigen and a boosting preparation comprising the antigen and liposomes (antigen-liposome preparation) (heterologous immunization), thereby eliciting the desired immune response in the animal), is also described.

The desired immune response is against a pathogen and the antigen is a pathogen-specific antigen.

The priming preparation is administered by a route selected from: subcutaneously, intramuscularly, intradermally and mucosally, and the boosting preparation is administered by a route selected from: subcutaneously, intramuscularly, intradermally and mucosally.

A method wherein priming preparation for heterologous immunization is admmistered intramuscularly to said animal, and wherein said boosting preparation is administered intranasally to said animal, is also described.

The boosting preparation comprises a pathogen-specific protein antigen encapsulated in liposome.

The liposomes in the boosting preparation average about 0.5-5 $\mu$m in size.

Each of the priming preparation and the boosting preparation, when administered alone to the animal, is insufficient to accomplish the desired level of immune response.

The immune response confers immunity against the pathogen.

The priming comprises one or two administrations separated by about 3-6 weeks, and wherein said boosting preparation is administered about 3 to 10 weeks following the last priming.

The priming comprises one or two administrations separated by 3 days, wherein said boosting preparation is administered 3 weeks following the last priming.

Liposomes in said antigen-liposome preparation average about 0.5-5 $\mu$m in size.

The boost dose is administered intranasally to said animal to induce IgA response.

The IgA response includes systemic and mucosal response.

A method for eliciting an immune response biased towards a Th2 response in an animal. The method comprises administering a priming preparation and a boosting preparation, each of which comprises the same antigen and liposomes (homologous immunization), is also described.

The host animal is a mammal, such as human or a non-human animal, including a domestic livestock animal (cow, pig, horse, or sheep, etc.), a pet (dog or cat, etc.), or a small experimental animal. (mouse, or rat, etc.).

The pathogen is a virus, such as Hepatitis-B virus (HBV).

Optionnally the pathogen is one of the strains of Chlamydia.
Optionnally the pathogen is a bacterial strain, such as Bacillus anthracis.
Optionnally the pathogen specific antigen is a protective antigens (PA), such as the PA antigen of Bacillus anthracis.
Optionnally the entrapped immunogen in liposome is a peptide that activates T cells or a inactivated whole virus particle.
Optionnally the pathogen-specific antigen is a viral surface antigen, such as HBsAg.
Optionnally the pathogen-specific antigen is a peptide.
Optionnally the pathogen-specific antigen is a kind of inactivated virus or bacterium.
Optionnally the pathogen-specific antigen (peptide, protein or inactivated virus) is encapsulated in liposomes.
Optionnally the pathogen-specific antigen (peptide, protein or inactivated virus/bacterium) is mixed with empty liposomes.
Optionnally the antigen-liposome preparation is freshly prepared.
Optionnally the antigen-liposome preparation is lyophilized before use.
Optionnally the method further comprises administering one or more boosting preparation of the same said antigen-liposome preparation (homologous immunization), wherein each administration is separated by about 2-16 weeks, preferably about 3-6 weeks.
Optionnally all preparations have the same amount of said pathogen-specific antigen.
Optionnally different amount of priming and boosting are administered to said animal via the same route.
Optionnally different amount of priming and boosting preparations are administered to said animal via separate routes.
Optionnally the boosting preparation(s) and the initial priming are administered to said animal via the same route.
Optionnally the boosting preparation and the initial priming are administered to said animal via separate routes.
Optionnally the method further comprises measuring humoral and/or cellular immune responses to said pathogen-specific antigen.
Optionnally the humoral immune response includes total antigen specific antibody (Ig) titers in serum or at mucosal surfaces; liters of anti-HBsAg-specific antibodies in serum or at mucosal surfaces; titers of antigen specific antibody isotypes and/or sub-types including IgG, IgA, IgG1, and IgG2a; ratio of IgG1 and IgG2a.
Optionnally the cellular immune response includes the appearance of $CD8^+$ IFN-$\gamma^+$ cytotoxic T cells (CTLs); enhanced CTL killing activity; secretion of cytokines characteristic of Th1 response including IFN-$\gamma$; secretion of cytokines characteristic or Th2 response including IL-10 and IL-4; and polarization of the T-helper cell profile (e.g., Th1 versus Th2 response).
Optionnally the humoral and/or cellular immune responses are generally measured from samples obtained from said host animals about 2, 4, 6 or 8 weeks post the last boost. However responses may be measured for many months after the last boost.
Optionnally the immune response includes IgA, IgG and T cell responses.
Optionnally the IgA response includes systemic and/or mucosal IgA response(s).
Optionnally the IgG response includes systemic and/or mucosal IgG response(s).
An immunogenic composition comprising two immunizing components, wherein the first immunizing component comprises a nucleic acid encoding an antigen and the second immunizing component comprises the antigen and liposome is also described. Optionnally the desired immune response is against a pathogen and the antigen is a pathogen-specific antigen.

## Brief Description of the Drawings

*Figure 1* shows the size distribution profile of liposomes sized to an average of 4 μm in diameter. The x-axis shows size in μm and the y-axis shows particle number.

*Figure 2* is representative of the degree of variation in serum HBsAg-specific Ig titers that is observed between individual CD1 (top panel) and Balb/c (bottom panel) mice within an experiment. Responses are shown from mice that received one (closed circles) or two (closed triangles) homologous immunizations with HBsAg-liposomes IN, HBsAg- liposomes IM, or the GSK commercial vaccine for HBsAg IM. End- point titers are defined as a serum titer >2x that observed for naive mice. Horizontal lines denote the average response (n= 5 mice per group).

*Figure 3* illustrates the kinetics of HBsAg-specific serum Ig levels in CD1 mice after intranasal immunization with HBsAg-liposomes. The data are shown as the OD at 450 nm in the HBsAg-specific Ig ELISA assay after serial dilution of serum samples at various times after primary immunization (closed symbols) and at two weeks following a booster immunization (closed symbols with cross-hatches, +). Values are averages from serum pools (n= 5 mice per group). Responses to the GSK HBsAg vaccine are shown for comparison.

*Figure 4* illustrates the HBsAg-liposome dose response in CD1 mice immunized intranasally on week 0 (closed symbols) or on weeks 0 and 6 (closed symbols with cross-hatches, +). Responses to the GSK HBsAg vaccine are shown for comparison. HBsAg-specific serum Ig levels were measured on serum sample pools taken 8 weeks after first immunization (n= 5 mice per group).

*Figure 5* shows the effect of route of administration and delivery of a booster immunization on total HBsAg-specific serum antibody in CD1 mice administered HBsAg-liposomes (15 μg/dose). Closed symbols show responses in mice (n = 5) that received a single priming immunization. Closed symbols with cross-hatches (+) show responses in mice (n = 5) that received identical homologous primary and secondary booster immunizations. Responses to the GSK HBsAg vaccine are shown for comparison.

*Figure 6* shows the effect of route of administration and delivery of a booster immunization on total HBsAg-specific serum antibody in Balb/c mice administered HBsAg-liposomes (15 μg/dose). Closed symbols show responses in mice (n = 5) that received a single priming immunization. Closed symbols with cross-hatches (+) show responses in mice (n = 5) that received homologous primary and secondary booster immunizations. Responses to the GSK HBsAg vaccine are shown for comparison.

*Figure 7* shows a comparison of HBsAg-specific serum Ig responses in CD1 mice immunized intranasally with either fresh (squares) or lyophilized (triangles) HBsAg-liposomes, or immunized intramuscularly with the commercial GSK vaccine (circles). Closed symbols show responses in mice (n = 5) that received a single priming immunization. Closed symbols with cross-hatches (+) show responses in mice (n = 5) that received homologous primary and secondary booster immunizations. Total HBsAg-specific serum antibody responses were measured in the sera 8 weeks after the primary immunization.

*Figure 8* shows a comparison of HBsAg-specific serum Ig responses in CD1 mice immunized intramuscularly with either fresh (squares) or lyophilized (triangles) HBsAg-liposomes, or immunized intramuscularly with the commercial GSK vaccine (circles). Closed symbols show responses in mice (n = 5) that received a single priming immunization. Closed symbols with cross-hatches (+) show responses in mice (n = 5) that received homologous primary and secondary booster immunizations. Total HBsAg-specific serum antibody responses were measured in the sera 8 weeks after the primary immunization.

*Figure 9* shows the effect of liposome size on serum HBsAg-specific IgG responses in Balb/c mice (6 weeks after primary immunization and two weeks post boost). Liposomes containing HBsAg (15 μm) were sized at 4 μm, 1 μm and 0.2 μm and were administered intranasally to Balb/c mice (n = 5) as a single size, or as an equal mixture of all three sizes. A quantitative ELISA for HBsAg-specific IgG was used to determine serum IgG levels in pooled serum samples (n= 5 mice per group). The vertical bar in the mix group shows the predicted value if the antibody responses are additive.

*Figure 10* shows IgG1:IgG2a ratios of HBsAg-specific antibodies in the serum of CD1 mice immunized by different protocols. Grey bars are the IgG1:IgG2a ratios from mice that received one immunization on week 0. Black bars are the IgG1:IgG2a ratios from mice that received two homologous immunizations on weeks 0 and 6. Quantitative HBsAg- specific ELISA assays were used to determine the level of HBsAg- specific IgG1 and IgG2a in the serum from a serum pool (equal amounts of serum from 4-5 individual mice per group) 8 weeks after the primary immunization. Two vertical lines at 0.5 and at 2.0 in each panel demarcate three different patterns of antibody responses. A ratio of 0.5 or less indicates a Th1 polarized response. A ratio of 2.0 or more indicates a Th2 polarized response. Ratios between 0.5 and 2.0 indicate a mixed, or balanced response.

*Figure 11* shows IgG1:IgG2a ratios of HBsAg-specific antibodies in the serum of Balb/c mice immunized by different protocols. Grey bars are the IgG1:IgG2a ratios from mice that received one immunization on week 0. Black bars are the IgG1:IgG2a ratios from mice that received two homologous immunizations on weeks 0 and 6. Quantitative HBsAg- specific ELISA assays were used to determine the level of HBsAg- specific IgG1 and IgG2a in the serum from a serum pool (equal amounts of serum from 4-5 individual mice per treatment group) 8 weeks after the primary immunization. Two vertical lines at 0.5 and at 2.0 in each panel demarcate three different patterns of antibody responses. A ratio of 0.5 or less indicates a Th1 polarized response. A ratio of 2.0 or more indicates a Th2 polarized response. Ratios between 0.5 and 2.0 indicate a mixed, or balanced, response.

*Figure 12* shows that HBsAg-DNA is a weak immunogen for antibody responses in CD1 (squares) and Balb/c (triangles) mice. Responses to the GSK vaccine in CD1 mice (filled circles with cross-hatches, ±) and in Balb/c mice (open circles with cross-hatches, +) are shown for comparison. Closed symbols show responses in mice (n = 5) that received a single priming immunization. Symbols with cross-hatches (+) show responses in mice (n = 5) that received homologous primary and secondary booster immunizations. Total HBsAg-specific serum antibody responses were measured in the sera 8 weeks after the primary immunization. Mice received either 100 μg of HBsAg-DNA or 3 μg of the alum-based protein HBsAg vaccine (GSK) IM.

*Figure 13* shows that IM immunization of Balb/c mice with HBsAg-DNA polarizes the peripheral T cell compartment (spleen cells) to a Th1- biased cytokine profile. The left panel shows results from the IFN-γ ELISPOT assay, and the right panel shows results from the IL-10 ELISPOT assay. The graph shows a comparison between HBsAg CTL peptide and HBsAg protein in activating T cells to cytokine production in both assays. Irrelevant peptide and protein are also included as specificity controls. Polyclonal responses to the T cell mitogen Concanavalin A (Con A) are shown for a maximum response for IL-10. Results for Con A stimulation in the IFN-γ assay are not shown since spots were too numerous to accurately quantify. Both peptide and protein are able to stimulate IFN-γ production in HBsAg-DNA immunized mice, but neither produces significant IL-10 production, directly demonstrating a strong Th1 cytokine bias in DNA immunized mice. Non-immunized mice failed to produce detectable numbers of spots to either cytokine on stimulation with the HBsAg peptide or protein (data not shown).

*Figure 14* illustrates the effect of heterologous immunization in Balb/c mice: HBsAg-DNA immunized mice produce high levels of HBsAg-specific serum Ig after boosting with a low dose of HBsAg-liposomes IN. Mice were immunized with HBsAg-DNA intramuscularly (100 μg on weeks 0 and 6) and boosted with a low dose of HBsAg-liposomes (3 μg/dose on week 12, n = 8 mice per group). Closed symbols show total HBsAg-specific serum Ig antibody responses prior to the IN boost. Closed symbols with cross hatches (+) show HBsAg-specific total serum Ig responses four weeks after the IN boost.

*Figure 15* illustrates the effect of heterologous immunization in Balb/c mice: HBsAg-DNA immunized mice produce high levels of HBsAg-specific serum and vaginal IgA after boosting with a low dose ofHBsAg- liposomes IN. Mice were immunized with HBsAg-DNA intramuscularly (100 μg on weeks 0 and 6) and boosted with a low dose of HBsAg- liposomes (3 μg/dose on week 12). Closed symbols show total HBsAg- specific IgA antibody responses just prior to the IN boost. Closed symbols with cross hatches (+) show HBsAg-specific IgA responses 4-5 weeks after the IN boost. As a positive control, serum IgA responses are shown for mice immunized homologously on weeks 0 and 6 with 15 μg of HBsAg-liposomes and tested two weeks later (circles with cross hatches, +). All groups contained 8 mice.

*Figure 16* shows that mucosal IgG is also generated in mice immunized with the heterologous immunization protocol. Balb/c mice received two IM priming immunizations with HBsAg-DNA (100 μg each) followed by IN immunization with either HBsAg-liposomes (15 μg), empty liposomes, or were left untreated (n=5 per group). Four weeks after the final immunization, mice were sacrificed and lung and vaginal washes were measured. HBsAg-specific IgG titers were obtained on sample pools using an antigen-specific sandwich ELISA assay. Only mice that received the combination of HBsAg-DNA and HBsAg-liposomes IN generated antigen-specific mucosal IgG responses above background levels observed in untreated mice.

*Figure 17* shows the IgG1: IgG2a ratios in the serum of individual Balb/c mice after a heterologous immunization protocol that consisted of priming IM with HBsAg-DNA (100 μg on weeks 0 and 6) and boosting with HBsAg-liposomes (3 μg on week 16). Serum samples were taken four weeks after the boost. All mice showed a Th1-biased immune response (IgG1: IgG2a ratio of $< 0.5$). These results indicate that the Th1-biased response that is seen after HBsAg-DNA immunization alone is preserved even after delivery of a HBsAg-liposomes, which promote Th2-biased responses in Balb/c mice (compare with Figure 11).

*Figure 18* illustrates HBsAg-specific serum IgG avidity indices in Balb/c mice immunized by homologous immunization protocols (left panel) or heterologous immunization protocols (right panel). Mice were immunized on weeks 0 and 4 (homologous immunization) or were primed on weeks 0 and 4, and boosted on week 8 (heterologous immunization). Antibody avidities were measured on sera obtained two weeks after the last immunization. The dashed line shows the maximum avidity values that are possible in this quantitative assay. The heterologous immunization protocol generates the highest avidity HBsAg-specific serum IgG antibodies. Homologous immunization with Ag-liposome IN from the same experiment is shown in the right panel for comparison.

*Figure 19* illustrates representative results from an *in vivo* CTL killing assay. Left panel: Spleen cells from naive Balb/c mice. Right panel: Spleen cells from Balb/c mice that received the heterologous immunization regimen (2x HBsAg-DNA IM + 1x HBsAg-liposomes IN). All mice received a mixture of $CFSE^{low}$ unpulsed syngeneic spleen cells and $CFSE^{high}$ HBsAg peptide pulsed syngeneic spleen cells. 20 hours later, recipient spleens were harvested and analyzed for CFSE fluorescence (x axis) by flow cytometry. Region 1 is gated on non-fluorescing (recipient) spleen cells. Region 2 is gated on $CFSE^{low}$ (unpulsed donor) cells. Region 3 is gated on $CFSE^{high}$ (HBsAg-peptide pulsed donor) cells. 200,000 viable cells were analyzed. The disappearance of $CFSE^{high}$ cells (R3) in the right panel indicates HBsAg peptide-specific killing of the transfused target cells by CTLs in the immunized mice.

*Figure 20* illustrates the clearance of HBsAg-vaccinia virus (W-HBsAg) from the lungs of mice that received the heterologous immunization regimen. Balb/c mice were immunized as indicated (treatment groups; n= 5 mice per group) at intervals of four weeks. Mice received doses of 100 $\mu$g of HBsAg-DNA IM and 15 $\mu$g of HBsAg-liposomes IN. Mice were infected 14 days after the boost with 2 x $10^5$ PFU of VV-HBsAg delivered in a 50 $\mu$l volume intranasally. Mice were sacrificed on day 5 after virus challenge, and viral titers were determined on lung homogenates using Vero cells. Values shown are the mean $\pm$ SD of four mice per group.

## Detailed Description of the Invention

I. Overview

The instant invention is at least partially based on Applicants' surprising discovery that combining heterologous immunization protocol with an antigen or an immunogen mixed with liposome successfully elicits a wide array of robust immune responses. Applicants have devised novel methods for eliciting desired immune responses to an antigen or immunogen. Two examples highlight the robust immune responses that the methods of the invention are able to elicit in an animal. First, the methods of the invention can be used to elicit immune responses that protect animals from challenges by live pathogens such as live virus. Second, vaccination using the methods of the invention conferred immunity to individuals who are non-responsive to currently-available vaccines. Furthermore, the novel methods provided herein achieve the desired immune responses without the need for any adjuvant other than liposome, thus avoiding risks and complications associated with many adjuvants, especially the bacterial toxins such as cholera toxin (CT) and the E. coli heat labile enterotoxin (LT). Depending on the particular immunization protocol used, the methods described herein are able to induce a wide range of immune responses that includes a combination of the following: robust antibody responses, T cell responses such as CTL generation, and Th1-type cytokine production and local immunity at mucosal sites.

Accordingly, the present invention provides a method for eliciting a desired immune response in an animal, comprising nucleic acids encoding an antigen and a boosting preparation comprising the antigen and liposomes to elicit the desired immune response in the animal. The method is particularly useful for eliciting an immune response in a host animal against a pathogen. In such context, the priming preparation comprises nucleic acid encoding a pathogen-specific antigen and the boosting preparation comprises the pathogen-specific antigen and liposomes. In certain embodiments, the pathogen is Hepatitis B Virus (HBV). The heterologous immunization regimens of the present invention includes priming and boosting with different forms of HBsAg vaccines (e.g., HBsAg-DNA IM and HBsAg-liposomes IN or IM) that result in more robust systemic immune responses and also the induction of local immunity at mucosal sites. In one embodiment, priming with HBsAg-DNA vaccine intramuscularly and boosting with HBsAg-liposomes intranasally induced potent synergistic antibody responses, activated CTLs and Th1-type cytokine profiles, and elicited the production of secretory IgA and IgG at mucosal sites. In another embodiment, priming with HBsAg-DNA vaccine intramuscularly and boosting with HBsAg-liposomes intranasally generated substantial antigen-specific lung CTLs and complete virus clearance following intransasal challenge with a recombinant vaccinia virus expressing HBsAg (VV-HBsAg). Delivery of the boost by the intranasal route was established as an important determinant for the generation of local immunity.

The present invention also provides a kit for eliciting an immune response in a host animal. The kit comprises a first immunizing component comprising a nucleic acid sequence encoding an antigen, a second immunizing component comprising the antigen and liposomes, and an instruction for a user to administer to the animal the first immunizing component followed with administering the second immunizing component to elicit an immune response in the animal.

The invention further provides a method for modulating immune responses such that a desired immune response biased towards a Th1 response may be elicited in a host animal. The method comprises administering intramuscularly to the animal a priming preparation comprising a nucleic acid sequence encoding an antigen; and subsequently administering to the animal a boosting preparation comprising the antigen and liposome. "Biased towards" refers to the situation where the observed immune response is closer to a Th1 or Th2 response compared to before immunization. In certain embodiments, immunization will completely switch a Th2 response to a Th1 response. In other embodiments, immunization may not completely switch a Th2 response to a Th1 response, but instead, result in a mixed response or a weaker Th2 response.

The instant invention additionlly provides a method to develop effective vaccines and vaccination protocols to specifically target the most common entry portal for microorganisms, the mucosal surfaces of the body. The invention provides that vaccines could be made more effective by encapsulating them into liposomes with a defined composition / size, and by delivering them directly to mucosal surfaces. By combining liposome encapsulated vaccines with appropriate adjuvants and immunization regimens, immune responses could be tailored to provide more effective and specific vaccines. In certain embodiments, the ideal vaccine would generate a balanced or T helper 1 (Th1)-biased immune response that also includes robust antibody responses, CTL generation and Th1-type cytokine production, and local immunity at mucosal sites. In other embodiments, it may be possible to tailor the immune response to generate a T helper 2 (Th2)-biased immune response, which may be beneficial in preventing rejection in graft hosts and in protecting against certain parasitic infections.

The instant invention provides methods to determine the optimal conditions for the encapsulation of various antigens (such as HBsAg) into liposomes (e.g., dose of HBsAg, liposome size, liposome: protein ratio, and fresh versus lyophilized and reconstituted liposomes) to generate the most robust antibody responses. Serum antibody responses were compared in mice that received such encapsulated antigens in liposomes (HBsAg-liposome) by intranasal (IN) and intramuscular (IM) routes of delivery. The invention also discloses the effect of serum antibody response after one administration (prime) or two administrations (prime and boost) of the same form of antigen (homologous immunization). Liposomes were found to be both a very effective adjuvant and delivery vehicle for inducing immunity to HBsAg via the intranasal or intramuscular routes. In one embodiment, antibody responses for the test antigen HBsAg achieved or exceeded the levels observed in mice treated with the commercially available HBsAg vaccine (GlaxoSmithKline) currently used in humans. Targeted delivery of antigen to the mucosal surfaces of the nasal passages also stimulated the local production of secretory IgA in mucosal secretions at all mucosal surfaces sampled.

Thus the invention provides several vaccine delivery protocols which promote different types of immune responses (antibody production, T cell cytokine profiles, cellular immunity by CTLs, and local immunity at mucosal surfaces). Rational combinations of these delivery platforms allow for the development of vaccines that are tailored to provide the best protection against specific pathogens under specific circumstances, for example, to favor Th1 or Th2 type immune responses, or a mixed / more balanced response.

In one aspect, the present invention provides methods and reagents for effectively eliciting immune responses in animals, especially mammals, against certain antigens, such as viral antigens. One salient feature of the invention relates to the use of liposome-encapsulated protein antigens delivered intranasally (IN) or intramuscularly (IM) to the host animal.

In certain embodiments, the instant invention provides methods and reagents for generating more effective vaccines by encapsulating antigens into liposomes of a defined composition and size, and by delivering the resulting antigen-liposome preparation directly to the mucosal immune system. In other embodiments, application of the specific platform delivery technologies of the invention provides rational combination of such liposome-encapsulated vaccines with appropriate adjuvants and immunization regimens, to tailor immune responses so as to provide more effective and specific vaccines (enhancing cell mediated and humoral immune response, tailoring Th2 and mixed / balanced Th1/Th2 to Th1 response, increasing IFN-$\gamma$ secretion, induction of high level mucosal IgA, IgG and CTL).

## II. Immunization Protocols

One component of the methods and kits of the present invention is the use of a "priming" immunization, comprising the initial administration of one or more antigens to an animal, especially a human patient, in preparation for subsequent administration(s) of the same antigen. Specifically, the term "priming", as used herein, refers to a first immunization using an antigen which induces an immune response to the desired antigen and recalls a higher level of immune response to the desired antigen upon subsequent reimmunization with the same antigen when administered in the context of the same or a different vaccine delivery system.

Another component of the methods and kits of the present invention is the use of a "boosting immunization", or a "boost", which means the administration of a composition delivering the same antigen as encoded in the priming immunization. A boost is sometimes referred to as an anamnestic response, i.e. an immune response in a previously sensitized animal. Multiple boosts can be administered, utilizing the same or differing amounts for each boost. A boosting that uses an antigen-delivery system different from the priming can be referred to as a "Heterologous boost", whereas a boosting that uses the same antigen-delivery system as the priming can be referred to as a "homologous boost."

As an alternative to sequential administration as described above, the priming preparation and the boosting preparation may be administered simultaneously, i.e. at substantially the same time. The methods of the invention lead to potent synergistic effects between the priming immunization and the boosting immunization in terms of immune responses the methods are able to elicit in an animal. As a result, the methods of the invention enable one to elicit a desired level of immune response, where each of the priming preparation and the boosting preparation, when administered alone to the animal, is insufficient to accomplish the desired level of immune response. In certain particular embodiments, heterologous immunization with intranasal boost generates mucosal CTL ($CD8^+$ $IFN\gamma^+$ phenotype and *in vivo* CTL killing), and provides complete protection against live pathogen challenge, such as challenges by live HBV as shown by Applicants in the illustrative examples.

The effects of immune response in the host animal can be assessed by various assays, including humoral and cellular immune responses. Humoral immune response includes total antigen-specific antibody (Ig) titers in serum or at mucosal surfaces; titers of HBsAg-specific antibodies in serum or at mucosal surfaces; titers of specific antibody isotypes and/or sub-types including IgG, IgA, IgG1, and IgG2a; ratio of IgG1 and IgG2a. Cellular immune response includes cytotoxic T cell (CTL) phenotype and activity. ; Cellular immune response also includes secretion of cytokines characteristic of Th1 responses including IFN-γ, and secretion of cytokines characteristic of Th2 response including IL-10 and IL-4. The cytokines are detected directly by cytokine ELISPOT assays (ie, IFN-γ and IL-10) and inferred from IgG1 : IgG2a ratios (e.g., Th1 versus Th2 response).

Each of the priming preparation and the boosting preparation may be administered by any one of the following routes: subcutaneously, intramuscularly, intradermally and mucosally. The priming preparation and the boosting preparation may be administered by the same route, or by different routes. In certain preferred embodiments, the priming preparation is administered intramuscularly and the boosting preparation is administered either intramuscularly or intranasally, which results in not only robust systemic immune responses but also the induction of local immunity at mucosal sites. In one particular embodiment, priming with HBsAg-DNA vaccine intramuscularly and boosting with HBsAg-liposomes intranasally induced potent synergistic antibody responses, activated CTLs and Th1-type cytokine profiles, and preserved secretory IgA production at mucosal sites. Delivery of the boost by the intranasal route was established as an important determinant for the generation of local immunity.

Different intervals between the priming administration and the boosting administration may be used. The boost administration can be done 2-8 weeks, preferably 4-6 weeks apart from the previous administration (initial or a prior boost). Typically, one boost administered 4-6 weeks after the initial administration is sufficient. The invention contemplates that either the priming preparation or the boosting preparation, or both, may be administered to the animal one or more times.

The boost administration(s) and the initial administration(s) may use the same or different amounts of antigen-liposome preparations, and the boost and the initial administrations can be administered via the same or different routes. The initial administration(s) and the boost administration(s) may use different amounts of DNA antigen delivered according to different schedules, and different amounts of antigen-liposome preparations delivered.

## III. Antigens

As used herein, the terms "antigen" or "immunogen", used interchangeably, are intended to encompass all peptide or protein sequences which are capable of inducing an immune response within the animal concerned. The terms "antigen" or "immunogen" encompass peptide or protein analogs of known or wild-type antigens, which analogs may be more soluble or more stable than wild type antigen, and which may also contain mutations or modifications rendering the antigen more immunologically active or optimized for expression in certain cell types (ie. humanized codon usage). An antigen may also be a peptide in which particular amino acid substitutions have been made to a naturally-occuring antigen that alter protein structure, a portion of the naturally-occuring antigen including known protective epitopes (ie. CTL epitopes), or a synthetically derived string of known epitopes that may or may not be limited to one pathogen (multivalent vaccine).

Further peptides or proteins that have sequences homologous with a desired antigen's amino acid sequence, where the homologous antigen induces an immune response to the respective pathogen, are also useful. Genes that are homologous to the desired antigen-encoding sequence should be construed to be included in the instant invention provided they encode a protein or polypeptide having a biological activity substantially similar to that of the desired antigen.

Analogs of the antigens described herein can differ from naturally occurring proteins or peptides by conservative amino acid, sequence differences or through modifications that do not affect sequence, or by both. For example, conservative amino acid changes may be made, which although they alter the primary sequence of the protein or peptide, do not normally alter its function. Modifications (which do not normally alter primary sequence) include in vivo, or in vitro chemical derivatization of polypeptides, e.g., acetylation, or carboxylation. Also included as antigens are proteins modified by glycosylation, e.g., those made by modifying the glycosylation patterns of a polypeptide during its synthesis and processing or in further processing steps; e.g., by exposing the polypeptide to enzymes which affect glycosylation, e.g., mammalian glycosylating or deglycosylating enzymes. Also included as antigens according to this invention are sequences which have phosphorylated amino acid residues, e.g., phosphotyrosine, phosphoserine, or phosphothreonine. Also included as antigens are polypeptides that have been modified using ordinary molecular biological techniques so as to improve their resistance to proteolytic degradation or to optimize solubility properties. Analogs of such polypeptides include those containing residues other than naturally occurring L-amino acids, e.g., D-amino acids or non-naturally occurring synthetic amino acids. The antigens of the invention are not limited to products of any of the specific exemplary processes listed herein.

An antigen can be a full-length antigen, an immunogenic fragment thereof, or an epitope derived from the antigen. In certain embodiments, the pathogen-specific antigen in the boosting preparation may be in the form of an attenuated or killed pathogen. Effective antigens also include surface antigens of these pathogens.

The term "epitope" as used herein refers to a sequence of at least about 3 to 5, preferably about 5 to 10 or 15, and not more than about 1,000 amino acids (or any integer therebetween), which define a sequence that by itself or as part of a larger sequence, binds to an antibody generated in response to such sequence or stimulates a cellular immune response. The term "epitope" encompasses sequences identical to the native sequence, as well as modifications to the native sequence, such as deletions, additions and substitutions (generally conservative in nature). The antigens used in the invention may comprise only a single epitope, such as, for example, a single CTL epitope.

The antigens encoded by the nucleic acids in the priming preparation and the antigens in the boosting preparation preferably have overlapping epitopes. In certain embodiments, the two antigens may be identical to each other. Alternatively, the two antigens may have overlapping but different set of epitopes. By way of an illustrating example, in a vaccination protocol for HBV, HBsAg-DNA may be used in the priming preparation, and the boosting preparation may be inactivated HBV. By way of another illustrating example, the priming preparation may be a minigene encoding a single epitope found in HBsAg, and the boosting preparation may comprise HBsAg antigen, or vice versa.

The following are illustrative examples of antigens that may be used in the present invention.

The antigens may be derived from HIV-1, (such as tat, nef, gp120 or gp160, gp40, p24, gag, env, vif, vpr, vpu, rev), human herpes viruses such as gH, gL gM gB gC gK gE or gD or derivatives thereof or Immediate Early protein such as ICP27, ICP 47, IC P 4, ICP36 from HSV1 or HSV2, cytomegalovirus, especially Human, (such as gB or derivatives thereof), Epstein Barr virus (such as gp350 or derivatives thereof), Varicella Zoster Virus (such as gpI, II, III and IE63), or from a hepatitis virus such as hepatitis B virus (for example Hepatitis B Surface antigen or Hepatitis core antigen or pol), hepatitis C virus antigen and hepatitis E virus antigen, or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18, eg L1, L2, E1, E2, E3, E4, E5, E6, E7), flaviviruses (e.g. Yellow Fever Virus, Dengue Virus, Tick-borne encephalitis virus, Japanese Encephalitis Virus) or Influenza virus cells such as HA, NP, NA, or M proteins, or combinations thereof), or antigens derived from bacterial pathogens such as *Neisseria spp,* including *N. gonorrhea* and *N. meningitidis* eg, transferrin-binding proteins, lactoferrin binding proteins, PilC, adhesins); *S. pyogenes* (for example M proteins or fragments thereof, C5A protease), *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M tuberculosis* (for example ESAT6, Antigen 85A, -B or -C, MPT 44, MPT59, MPT45, HSP10, HSP65, HSP70, HSP 75, HSP90, PPD 19kDa [Rv3763], PPD 38kDa [Rv0934]), *M. bovis, M leprae, M avium, M paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E. coli* (for example shiga toxin-like toxin or derivatives thereof); *Vibrio spp,* including *V. cholera* (for example cholera toxin or derivatives thereof); *Shigella spp,* including *S. sonnei, S. dysenteriae, S. flexnerii; Yersinia spp,* including Y. enterocolitica (for example a Yop protein), *Y. pestis, Y. pseudotuberculosis; Campylobacter spp,* including *C. jejuni* (for example toxins, adhesins and invasins) and *C. coli; Salmonella spp,* including *S. typhi, S. paratyphi, S. choleraesuis, S. enteritidis; Listeria spp.,* including *L. monocytogenes; Helicobacter spp,* including *H. pylori* (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C.* tetani (for example tetanus toxin and derivative thereof), *C. botulinum* (for example botulinum toxin and derivative thereof), *C. difficile* (for example clostridium toxins A or B and derivatives thereof); *Bacillus spp.,* including *B. anthracis* (for example botulinum toxin and derivatives thereof); *Corynebacterium spp.,* including *C. diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia*

*spp.,* including *E. equi* and the agent of the Human Granulocytic Ehrlichiosis; *Rickettsia spp,* including *R. rickettsii; Chlamydia spp., including C. trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii* (for example SAG2, SAG3, Tg34); *Entamoeba spp.,* including *E. histolytica; Babesia spp.,* including *B. microti; Trypanosoma spp.,* including *T. cruzi; Giardia spp.,* including *G. lamblia; Leshmania spp.,* including *L. major; Pneumocystis spp.,* including *P. carinii; Trichomonas spp.,* including *T. vaginalis; Schisostoma spp.,* including *S. mansoni,* or derived from yeast such as *Candida spp.,* including *C. albicans; Cryptococcus spp.,* including *C. neoformans.*

Other preferred specific antigens for *M. tuberculosis* are for example Rv2557, Rv2558, RPFs: Rv0837c, Rv1884c, Rv2389c, Rv2450, Rv1009, aceA (Rv0467), PstS1, (Rv0932), SodA (Rv3846), Rv2031c 16kDa1., Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSI-mTCC2, Erd14-DPV-MTI-MSL, DPV-MTI-MSL-mTCC2, TbH9-DPV-MTI (WO 99/51748). Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S. pneumoniae* (PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy variants or fusion proteins thereof.

The antigens that may be used in the present invention may further comprise antigens derived from parasites that cause Malaria. For example, preferred antigens from *Plasmodia falciparum* include RTS,S and TRAP.RTS is a hybrid protein comprising substantially all the C-terminal portion of the circumsporozoite (CS) protein *of P-falciparum* linked via four amino acids of the preS2 portion of Hepatitis B surface antigen to the surface (S) antigen of hepatitis B virus. Its full structure is disclosed in the International Patent Application No. PCT/EP92/02591, published under Number WO 93/10152 claiming priority from UK patent application No. 9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMPI, Pf332, LSA1, LSA3, STARP, SALSA, PfEXP1, Pfs25, Pfs28, PFS27/25, Pfs16, Pfs48/45, Pfs230 and their analogues in Plasmodium spp.

Vaccines of the present invention may also be used for the prophylaxis or therapy of chronic disorders in addition to allergy or infectious diseases. Such chronic disorders are diseases such as asthma, atherosclerosis, and Alzheimers and other autoimmune disorders. Vaccines for use as a contraceptive may also be considered.

Antigens relevant for the prophylaxis and the therapy of patients susceptible to or suffering from Alzheimer neurodegenerative disease are, in particular, the N terminal 39-43 amino acid fragment (ABthe amyloid precursor protein and smaller fragments. This antigen is disclosed in the International Patent Application No. WO 99/27944 - (Athena Neurosciences).

Potential self-antigens that could be included as vaccines for auto-immune disorders or as a contraceptive vaccine include: cytokines, hormones, growth factors or extracellular proteins, more preferably a 4-helical cytokine, most preferably IL13. Cytokines include, for example, IL1, IL2, IL3, IL4, IL5, IL6, IL7, IL8, IL9, IL10, IL11, IL12, IL13, IL14, IL15, IL16, IL17, IL18, IL20, IL21, TNF, TGF, GMCSF, MCSF and OSM. 4-helical cytokines include IL2, IL3, IL4, IL5, IL13, GMCSF and MCSF. Hormones include, for example, luteinising hormone (LH), follicle stimulating hormone (FSH), chorionic gonadotropin (CG), VGF, GHrelin, agouti, agouti related protein and neuropeptide Y. Growth factors include, for example, VEGF.

IV. <u>Priming preparation and boosting preparation</u>

The nucleic acids used in the priming preparation may be RNA or DNA including genomic DNA, synthetic DNA or cDNA. Preferably the nucleotide sequence is a DNA sequence and most preferably, a cDNA sequence. In order to obtain expression of the antigenic peptide within mammalian cells, it is necessary for the nucleotide sequence encoding the antigenic peptide to be presented in an appropriate vector system. By "appropriate vector" as used herein is meant any vector that will enable the antigenic peptide to be expressed within a mammal in sufficient quantities to evoke an immune response.

For example, the vector selected may be a plasmid, a phagemid or a viral vector. The vector may comprise promoter and polyadenylation/ transcriptional termination sequence arranged in the correct order to obtain expression of the antigenic peptides. The construction of vectors which include these components and optionally other components such as enhancers, restriction enzyme sites and selection genes, such as antibiotic resistance genes, is well known to persons skilled in the art and is explained in detail in Maniatis et al "Molecular Cloning: A Laboratory Manual", Cold Spring Harbour Laboratory, Cold Spring Harbour Press, Vols 1-3, 2nd Edition, 1989.

A vector carrying nucleic acids encoding an antigenic peptide can be administered in a variety of manners. It is possible for the vector to be administered in a naked form (that is as naked nucleotide sequence not in association with liposomal formulations, with viral vectors or transfection facilitating proteins) suspended in an appropriate medium, for example a buffered saline solution such as PBS and then injected intramuscularly, subcutaneously, intradermally or mucosally. It is additionally possible for the vectors to be encapsulated by, for example, liposomes or within polylactide co-glycolide (PLG) particles (25) for administration via the nasal or pulmonary routes.

It is also possible, according to one embodiment of the invention, for intradermal administration of the vector, preferably via use of gene-gun (particularly particle bombardment) administration techniques. Such techniques may involve coating of the vector on to gold beads which are then administered under high pressure into the epidermis, such as, for example, as described in Haynes et al J. Biotechnology 44: 37-42 (1996).

Recombinant viral vectors can also be used to deliver DNA antigens. Advantages to this approach include abundant expression of DNA-encoded proteins in multiple cell types, stronge enhancement of CTL responses and the ability of the virus to encode multiple genes. Vaccinia virus (including modified virus Ankara) and adenovirus (non-replicating) are two popular viruses used for vaccine development (Im and Hanke, Expert, Rev. Vaccines 3:S89-S97 (2004); Basak et al., Viral Immunol. 17:182-196 (2004)).

Recent modifications of DNA vaccines include the development of minigenes encoding single CTL epitopes, the use of gene-encoded targeting signals to allow more efficient presentation of epitopes by the MHC-pathway and the generation of secreted proteins to target MHC class II pathway (Doria-Rose and Haigwood, Methods 31:207-216 (2003); Leifert et al., Immunol. Rev. 199:40-53 (2004)).

The priming and boosting preparations are administered in such amount as will be prophylactically or therapeutically effective. The exact quantity may vary considerably depending on the species and weight of the animal being immunised, the route of administration, the potency and dose of the priming and boosting preparations, the nature of the disease state being treated or protected against, the capacity of the subject's immune system to produce an immune response and the degree of protection or therapeutic efficacy desired. Based upon these variables, a medical or veterinary practitioner will readily be able to determine the appropriate dosage level.

It is contemplated that the methods and kits of the invention can also be practiced with the addition of one or more adjuvants known in the art. An adjuvant is a substance or procedure which augments specific immune responses to antigens by modulating the activity of immune cells. Exemplary adjuvants include salt based adjuvants such as alum salts, bacterial-derived adjuvants like lipopolysaccharides and bacterial toxins, adjuvant emulsions that enable the slow release of antigen, agonsitic antibodies to co-stimulatory molecules, Freunds adjuvant, muramyl dipeptides, and recombinant/synthetic adjuvants. Alum is the most common salt-based adjuvant used to stimulate immune responses to protein vaccines and is the only adjuvant approved for human use in the United States (Alving, Vaccine 20(3):556-S64 (2002); Hunter, Vaccine 20(3):S7-12 (2002)). However, alum favors Th2-biased responses and does not stimulate cell-mediated immunity. Mucosal immunity can be induced through the use of bacterial toxins such as cholera toxin (CT) and the E. coli heat labile enterotoxin (LT), however the safety of these adjuvants is questionable (Alving, Vaccine 20(3):S56-S64 (2002); Hunter, Vaccine 20(3):S7-12 (2002)). The development of newer, safer adjuvants has recently focused on stimulating particular immune response pathways. Co-administration of cytokines, such as interferon-γ and granulocyte-macrophage colony stimulating factor (GM-CSF), has shown promise in stimulating cellular immune responses (reviewed in (Petrovsky and Aguilar, Immunol. Cell Biol. 82:488-496 (2004)). High levels of cytokines can cause toxicity however, and dosing regimens must be carefully modulated. Administration of cytokines has particular promise for DNA vaccination where genes encoding both the cytokine and antigen could be simultaneously expressed by the same vector. Additional adjuvants being explored include those that target the toll signaling pathway. CpG DNA motifs commonly found in bacterial DNA are potent activators of cellular immune responses, and newer generation DNA-based vaccines often encode multiple CpG motifs (reviewed in (Petrovsky and Aguilar, Immunol. Cell Biol. 82:488-496 (2004)).

V. Pathogens

The term "pathogen", as used herein, refers to any virus, bacteria and parasites that are capable of causing an infection in a host animal.

The instant invention can be used to effectively immunize host animals against a range of pathogens. In view of the teachings herein, one of skill in the art will understand that the methods and compositions of the invention are applicable to a wide range of viruses such as for example retroid viruses, RNA viruses, DNA viruses and envelop viruses. In one embodiment, the present invention is applicable to retroid viruses. In another embodiment, the present invention is further applicable to retroviruses (retroviridae). In yet another embodiment, the present invention is applicable to lentivirus, including primate lentivirus group. In a further embodiment, the present invention is applicable to Human Immunodeficiency virus (HIV), Human Immunodeficiency virus type-1 (HIV-1), Hepatitis B Virus (HBV), Hepatitis A Virus (HAV), Hepatitis C Virus (HCV), and Human T-cell Leukemia Virus (HTLV).

While not intended to be limiting, relevant retroviruses include: C-type retrovirus which causes lymphosarcoma in Northern Pike, the C-type retrovirus which infects mink, the caprine lentivirus which infects sheep, the Equine Infectious Anemia Virus (EIAV), the C-type retrovirus which infects pigs, the Avian Leukosis Sarcoma Virus (ALSV), the Feline Leukemia Virus (FeLV), the Feline Aids Virus, the Bovine Leukemia Virus (BLV), the Simian Leukemia Virus (SLV), the Simian Immunodeficiency Virus (SIV), the Human T-cell Leukemia Virus type-I (HTLV-I), the Human T-cell Leukemia Virus type-II (HTLV-II), Human Immunodeficiency virus type-2 (HIV-2) and Human Immunodeficiency virus type-1 (HIV-1).

The method and compositions of the present invention are further applicable to RNA viruses, including ssRNA negative-strand viruses and ssRNA positive-strand viruses. The ssRNA positive-strand viruses include Hepatitis C Virus (HCV). In a preferred embodiment, the present invention is applicable to mononegavirales, including filoviruses. Filoviruses further include Ebola viruses and Marburg viruses. Other RNA viruses include picomaviruses such as enterovirus, poliovirus, coxsackievirus and hepatitis A virus, the caliciviruses, including Norwalk-like viruses, the rhabdoviruses, including rabies virus, the togaviruses including alphaviruses, Semliki Forest virus, denguevirus, yellow fever virus and rubella virus, the orthomyxoviruses, including Type A, B, and C influenza viruses, rotavirus, the bunyaviruses, including the Rift Valley fever virus and the hantavirus, the filoviruses such as Ebola virus and Marburg virus, and the paramyxoviruses, including mumps virus and measles virus. Other viruses that can be treated with the methods of the present invention include Herpes Simplex type I viruses (HSV1), Herpes Simplex type II viruses (HSV2), Bacillus anthracis, Respiratory Syncytial Virus, Coronavirus (pathogen for SARS) and Foot-mouth disease virus (FMDV). Additional pathogens that can be treated include Chlamydia and parasites that cause malaria (including Plasmodium falciparum, Plasmodium vivax, Plasmodium malariae, and Plasmodium ovale).

The methods and kits according to the present invention can be used in relation to prophylactic or treatment procedures of all animals including, for example, domestic animals, laboratory animals, farm animals, captive wild animals and, most preferably, humans.

The methods and the kits of the invention are useful for therapeutic vaccination. The goal of therapeutic vaccination is to target immune responses in an infected individual to eradicate cells already infected with virus. Viruses that establish chronic infections without stimulating strong immune responses require the generation of novel therapeutic vaccination strategies (reviewed in (Berzofsky et al., J. Clin. Invest. 114:450-462 (2004)). Human papilloma virus (HBV), the hepatitis B and C viruses (HBV, HCV), herpes viruses and human immunodeficiency virus (HIV) are a number of viruses that establish long-term infections. The development of therapeutic vaccines for viruses has focused on the activation of CTL to recognize and destroy infected cells. Applicants have shown the methods of the invention are effective in enhancing cellular immune responses, making them suitable for providing therapeutic vaccination. The effectiveness of the methods may be further enhanced by inclusion of cytokine adjuvants and CpG motifs that have been shown to be particularly promising in the development of anti-cancer vaccines (Belardelli et al., Cancer Res. 64: 6827-6830 (2004)).

The vaccines of the present invention are particularly suited for the immunotherapeutic treatment of diseases, such as chronic conditions, but also for the therapy of persistent infections. Accordingly the vaccines of the present invention are particularly suitable for the immunotherapy of infectious diseases, such as Tuberculosis (TB), HIV infections such as AIDS and HBV infections.

VI. Liposome Preparation

The instant invention also provides a liposome based antigen delivery platform technology for vaccine applications via mucosal sites. The invention combines a liposome encapsulation technology, specific delivery to mucosal sites, and the use of adjuvants and heterologous immunization protocols to achieve a more balanced or mixed Th response.

Liposomes have several potential advantages as delivery platforms for vaccines. Encapsulation of antigens within liposomes sequesters these antigens, thus the liposomes serve as an antigen depot capable of sustained antigen release. In addition, liposomes are biocompatible and biodegradable, and low in toxicity and immunogenicity. When appropriately sized (e.g., > 0.2 μm up to 5 μm), liposomes are selectively taken up by antigen-presenting cells in the body, and have the potential to induce both humoral antibody and CTL responses. Liposomes serve as antigen carrier /vehicle as well as being an adjuvant that can be administered repeatedly without toxicity or immunogenicity.

Liposomes are structures consisting of a membrane bilayer composed of phospholipids of biological or synthetic origin, usually spherical in shape. Liposomes form naturally when phospholipids or lipids contact water. The structure of liposomes can be manipulated by methods to form them in the laboratory, including the input of energy in the form of heat, sonic energy, freeze-thaw cycles, or shear forces. Because liposomes have features of biological membranes, they can be engineered in the laboratory to contain a variety of biologically and therapeutic relevant complex molecules, including proteins. The phospholipid bilayer membrane of liposomes separates and protects entrapped materials in the inner aqueous core from the outside. Both water-soluble and -insoluble substances can be entrapped in different compartments, the aqueous core and bilayer membrane, respectively, of the same liposome. Chemical and physical interaction of these substances can be eliminated because the substances are in these different compartments.

Liposomes used with the methods and kits of the present invention can be prepared using any methods known in the art. These liposomes may have an average diameter of about 0.5 -5 microns, although liposomes of 0.2-8 microns may also be useful. In certain embodiments, liposomes that may be used in the methods and kits of the invention are "long circulating liposomes" (a.k.a. "sterically stabilized liposomes"), which are liposomes that comprise one or more specialized lipids that, when incorporated into liposomes, result in enhanced circulation lifetimes relative to liposomes lacking such specialized lipids. Examples of long circulating liposomes known in the art include those in which the liposome (A) comprises one or more glycolipids such as monosialoganglioside GM1 or (B) comprises one or more lipids derivatized with one or more hydrophilic polymers, such as a polyethylene glycol (PEG) moiety. While not wishing to be bound by any theory, at least for long circulating liposomes containing gangliosides, sphingomyelin, or PEG-derivatized lipids, the enhanced circulation half-life of these liposomes derives from a reduced uptake into cells of the reticuloendothelial system (RES) (Allen et al., FEBS Letters, 1987, 223, 42; Wu et al., Cancer Research, 1993, 53, 3765).

A. Glycolipid-Comprising Liposomes: Various liposomes comprising one or more glycolipids are known in the art. Papahadjopoulos et al. (Ann. N.Y. Acad. Sci., 1987, 507, 64) reported the ability of monosialoganglioside GM1, galactocerebroside sulfate and phosphatidylinositol to improve blood half-lives of liposomes. These findings were expounded upon by Gabizon et al. (Proc. Natl. Acad. Sci. USA, 1988, 85, 6949). U.S. Pat. No. 4,837,028 and published PCT application WO 88/04924, both to Allen et al., disclose liposomes comprising (1) sphingomyelin and (2) the ganglioside GM1 or a galactocerebroside sulfate ester. U.S. Pat. No. 5,543,152 (to Webb et al.) discloses liposomes comprising sphingomyelin. Liposomes comprising 1,2-sn-dimyristoylphosphatidylcholine are disclosed in published PCT application WO 97/13499 (to Lim et al.).

B. Liposomes Derivatized with Hydrophilic Polymers: Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, 2C1215G, that contains a PEG moiety. Illum et al. (FEBS Letters, 1984, 167, 79) noted that hydrophilic coating of polystyrene particles with polymeric glycols results in significantly enhanced blood half-lives. Synthetic phospholipids modified by the attachment of carboxylic groups of polyalkylene glycols (e.g., PEG) and liposomes comprising such phospholipids are described by Sears (U.S. Pat. Nos. 4,426,330 and 4,534,899). Klibanov et al. (FEBS Letts., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, e.g., DSPE-PEG, formed from the chemical attachment of PEG to DSPE (distearoylphosphatidylethanolamine).

Liposomes having covalently bound PEG moieties on their external surface are described in European Patent No. 0 445 131 B1 and published PCT application WO 90/04384 to Fisher. Liposome compositions containing 1-20 mole percent (mol %) of PE derivatized with PEG, and methods of use thereof, are described by Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in published PCT application WO 91/05545 and U.S. Pat. No. 5,225,212 (both to Martin et al.) and in published PCT application WO 94/20073 (Zalipsky et al.) Liposomes comprising PEG-modified ceramide lipids are described in published PCT application WO 96/10391 (Choi et al.). U.S. Pat. Nos. 5,540,935 (Miyazaki et al.) and 5,556,948 (Tagawa et al.) describe PEG-containing liposomes that can be further derivatized on their surfaces with functional moieties.

C. DMPG-Containing Liposomes: Various liposomes comprising dimyristoylphosphatidylglycerol (DMPG) have been described. Generally, however, such liposomes comprise DMPG in a mol % of about 10% or higher (see, for example, Akhtar et al. (Nucl. Acids Res., 1991, 19, 5551; Yachi et al. (Biopharm. Drug Dispos., 1996, 17, 699; and Farmer et al. (Meth. Enz., 1987, 149, 184). Liposomes having 3 mol % DMPG have been described, but such liposomes included a component (in particular, a phosphatidylcholine derivative) that is not found in the liposomal compositions of the present invention. Such phosphatidylcholine derivative components include, e.g., 10 mol % distearoylphosphatidylcholine (DSPC) (Brodt et al., Cancer Immunol. Immunother., 1989, 28, 54) or 7 mol % dimyristoylphosphatidylcholine (DMPC) (Perez-Soler et al., J. Nuclear Med., 1985, 26, 743; Wasan et al., Antimicrobial Agents and Chemotherapy, 1993, 37, 246; and Li et al., Oncology Res., 1995, 7, 611).

The liposome preparation may either be freshly prepared or lyophilized for long term storage. Both preparations can be used with comparable effectiveness. The liposomes used in the methods of the invention can be all the same (e.g. same compositions or same size), or include more than one types of liposomes.

In certain embodiments, commercially available liposomes can be used. For example, liposomes can be made under contract by Northern Lipids Inc. (Vancouver, BC), a Contract Research Organization that specializes in the development of lipid-based liposome formulations. In certain other embodiments, liposomes of various sizes can be prepared using the methodology as described below. The resulting liposomes, depending on specific preparation protocols, are typically sized at 4 μm, 1 μm, or 0.2 μm by passing preparations through a microfluidizer. Briefly, liposomes were prepared at the following lipid concentrations: Phosphatidylcholine / cholesterol / dicetyl phosphate 7/3/0.5 mole %. Antigen such as HBsAg is incorporated into multilaminar liposomes at several concentrations for the testing of immune responses. An average of 60% of the HBsAg was incorporated into the liposomes in the examples below. Liposome size was also measured with a N4 MC Submicron Particle Size Analyzer (Coulter Electronics). A representative profile of the 4 μm sized liposomes is shown in Figure 1. In one experiment, the liposome: protein ratio was decreased to 1/2 and 1/3 of the content usually used to encapsulate 15 μg of HBsAg. In certain embodiments, antigen preparations can be lyophilized for storage and then reconstituted before use. Both size and ζ-potential (zeta, a measure of charge) might be measured before use. The ζ-potential was determined using Zeta-Puls ζ-potential analyzer (Brookhaven Instruments). The lipid: antigen protein ratio can be varied in some preparations in order to determine the importance of this ratio on immune responses to the specific antigen (e.g., HBsAg).

**A. Basic method for liposomes with a size range of 2-4 μm.**

Liposomes of the subject invention were prepared at the following lipid concentrations: Phosphatidylcholine / cholesterol / dicetyl phosphate 7/3/0.5 mole %. Dicetyl phosphate was dissolved in chloroform plus 5% of ethanol, sonicated and phosphatidylcholine and cholesterol were then added. Lipids were dried in a Labconco rotary evaporator for one hour and traces of chloroform were removed by freeze-drying with a Freezone 4.5 Freeze Dry System overnight. The lipid film was hydrated with antigen, such as Hepatitis B surface antigen (HBsAg), at a concentration of 125-300 μg/ml in 10 mM HEPES-buffer, 150 mM NaCl, pH 7.4(HBS), and filtered with a 0.2μ m nylon filter. The mixture was vortexed thoroughly and allowed to sit for 1 hour and then vortexed again to ensure the formation of multilamellar vesicles. The resultant liposomes were then subjected to three cycles of freeze-and-thaw (1 cycle = freezing for one hour and thawing for one hour at room temperature). The size of the liposomes was measured with a N4 MD Submicron Particle Size Analyzer (Coulter Electronics). The ζ-potential was measured using Zeta-Puls ζ-potential analyzer (Brookhaven Instruments) in 5 mM HEPES buffer, 1.0 mM NaCl, pH 7.4.

**B. Liposomes with a size of 1.0 μm**

Liposomes of the subject invention were prepared at the following lipid concentrations: Phosphatidylcholine / cholesterol / dicetyl phosphate 7/3/0.5 mole %. Dicetyl phosphate was dissolved in chloroform plus 5% of ethanol, sonicated and phosphatidylcholine and cholesterol were then added. Lipids were dried in a Labconco rotary evaporator for one hour and traces of chloroform were removed by freeze-drying with a Freezone 4.5 Freeze Dry System overnight. The lipid film was hydrated with antigen (e.g., HBsAg) at a concentration of 125-300 μg/ml in 10 mM HEPES-buffer, 150 mM NaCl, pH 7.4, filtered with 0.2 μl nylon filter. The mixture was vortexed thoroughly and allowed to sit for 1 hour and then vortexed again to ensure the formation of multilamellar vesicles. The liposomes were then subjected to three cycles of freeze-and-thaw (1 cycle = freezing for one hour and thawing for one hour at room temperature). After the third cycle the liposomes were warmed in water bath to 50˚C and extruded through a polycarbonate filter with a pore size of 1.0 μm using hand-held Avanti-micro extruder. The size of the liposomes was measured as before.

**C. Liposomes with a size of 0.2 μm.**

Liposomes of the subject invention were prepared at the following lipid concentrations: Phosphatidylcholine / cholesterol / dicetyl phosphate 7/3/0.5 mole %. Dicetyl phosphate was dissolved in chloroform plus 5% of ethanol, sonicated and phosphatidylcholine and cholesterol were then added. Lipids were dried in a Labconco rotary evaporator for one hour and traces of chloroform were removed by freeze-drying with a Freezone 4.5 Freeze Dry System overnight. The lipid film was hydrated with antigen (e.g., HBsAg) at a concentration of 125-300 μg/ml in 10 mM HEPES-buffer, 150 mM NaCl, pH 7.4, filtered with 0.2 μm nylon filter. The mixture was vortexed thoroughly and allowed to sit for 1 hour and then vortexed again to ensure the formation of multilamellar vesicles. The liposomes were then subjected to three cycles of freeze-and-thaw (1 cycle = freezing for one hour and thawing for one hour at room temperature). After the third cycle, liposomes were warmed in a water bath to 50˚C and extruded first through a polycarbonate filter with a pore size of 1.0 μm, then with pore size of 0.4 μm and finally 0.2 μm using a hand-held Avanti micro-extruder. Then size of the liposomes was measured as previously.

**D. Preparation of lyophilized liposomes.**

Liposomes of the subject invention were prepared at the following lipid concentrations: Phosphatidylcholine / cholesterol / dicetyl Phosphate 7/3/0.5 mole %. Dicetyl phosphate was dissolved in chloroform plus 5% of ethanol, sonicated and phosphatidylcholine and cholesterol were then added. Lipids were dried in a Labconco rotary evaporator for one hour and traces of chloroform were removed by freeze-drying with a Freezone 4.5 Freeze Dry System overnight. The lipid film was hydrated with antigen (e.g., HBsAg) at a concentration of 125-300 μg/ml in HBS with 100 mg/ml maltose, filtered with 0.2 μm nylon filter. The mixture was vortexed thoroughly and allowed to sit for 1 hour and then vortexed again to ensure the formation of multilamellar vesicles. The liposomes were then subjected to three cycles of freeze-and-thaw (1 cycle = freezing for one hour and thawing for one hour at room temperature). The size of the liposomes was measured and liposomes were lyophilized with a Freezone 4.5 Freeze Dry System overnight. Lyophilized liposomes were reconstituted by vortexing with corresponding quantity of water to reach the needed antigen concentration.

VII. Assays

The following assays may be needed to carry out certain steps of the invention.

In general, as a skilled artisan is aware, many different but functionally equivalent assays may be used to achieve the same purpose. Thus none of the specifically described assays are limiting unless explicitly stated.

**A. Enzyme-Linked ImmunoSorbent Assays (ELISA)**

Anti-HBsAg specific antibody responses such as Ig, IgG, IgA, IgG1 and IgG2a in the collected samples of the immunized mice were tested by ELISAs. A three layer (antigen-test samples or Ig standards-HRP conjugated detecting antibody) ELISAs were designed for testing Ig, IgG, IgG1 and IgG2a antibody. 50 μl of 10 μg/ml of pure HBsAg(Advanced Immune Chemical) was coated in a 96 well ELISA plates. Serial dilutions of serum or secretory samples were added after blocking procedure. Goat anti-mouse Ig(H+L)-HRP, goat anti-mouse IgG-HRP, goat anti-mouse IgG1-HRP and goat anti-mouse IgG2a-HRP (SouthernBiotech) were used as detecting antiboies to test for anti-HBsAg specific Ig, IgG, IgG1, and IgG2a respectively in samples. Serially diluted IgG, IgG1 and IgG2a standards were used to generate standard curves for IgG, IgG1 and IgG2a antibody ELISAs respectively. Five layer capture ELISAs were used to test mucosal samples for mouse IgA and mouse IgG. 50 μl of a 4 μg/ml of rat anti-mouse IgA capture antibody was coated to the ELISA plates to capture IgA antibody from serum or mucosal (fecal, vaginal, lung wash and saliva) sites. 100 μl of 2 μg/ml of purified HBsAg was added to bind to anti-HBsAg specific IgA antibody. A goat anti-HBsAg is used to specifically bind to the captured HBsAg in the previous step. Donkey anti-goat IgA-HRP was the final detecting antibody before color development from the substrate-TMB. For the detection of mucosal IgG a rat anti-mouse IgG antibody was used as a capture antibody, followed by HBsAg, goat anti-HBsAg, and donkey anti-goat IgG-HRP at the same concentrations as used in the IgA ELISA.

**B. Measurement of IgG antibody avidities**

The avidity of HBsAg-specific IgG antibodies is evaluated using quantitative ELISA avidity assays. Assays are performed using optimized conditions for IgG antibodies, with the addition of the chaotropic reagent urea to disrupt antigen-antibody interactions. To insure consistent and accurate results, the relative amount of IgG in the sample dilutions tested is between 100-150 ng/ml, which is within the linear and quantitative portion of the standard curve. Diluted samples are first incubated on HBsAg-coated ELISA plates for 1 hour at room temperature to allow antigen-antibody binding to occur. Wells are washed, and subsequently incubated with standard ELISA buffer (PBS with 0.05% Tween-20 and 1% bovine serum albumin), or with varying concentrations of urea in ELISA buffer for 15 minutes at room temperature. After additional washes, the ELISA is carried out as usual. Avidity indices (AI) are calculated according to the following formula: ng/ml of IgG in the absence of urea/ ng/ml of IgG in the presence of urea.

**C. Detection of IFN-γ and IL-10 secreting cells by ELISPOT (Enzyme-Linked ImmunoSPOT) assays**

Spleen or peripheral blood cells from immunized mice and naïve were cultured *in vitro* for the measurement of IFN-γ and IL-10 secreting cells by ELISPOT assays. The ELISPOT assays were established using commercial antibody pairs and reagents (BD Pharmingen) and Multiscreen-IP multiwell ELISPOT plates (Millipore, Hopkington, MA). *In vitro* cultures were stimulated with HBsAg peptide at a concentration of 10 μg/2.5 x $10^6$ cells per ml for 20-22 hours. Cultures with no peptide were used as a negative control, and cultures stimulated with anti-CD3 or Con A were used as positive controls. IFN-γ and IL-10 ELISPOT plates from this study were quantitatively analyzed by Cellular Technology Ltd. (Cleveland, OH) for both frequencies of spots and spot size using a digital plate reader and software designed for this purpose.

### D. *In vivo* CTL cytotoxicity assays

*In vivo* CTL assays have been described recently in the literature (refs: Estcourt, M. J. et al. 2002. Int. Immunol. 14(1): 31-37; Barber, D. L. et al. 2003. J. Immunol. 171: 27-31; Kumaraguru, U. et al. 2004. J. Immunol. 172: 3719-3724) for measuring CTL activity in immunized animals. The *in vivo* CTL assay uses mice that have received two rounds ofHBsAg-DNA vaccine IM followed by the subject liposome preparation (e.g., HBsAg-liposome) IN. After the sample collection is completed for the measurement of antibody responses (six weeks or longer after the IN boost), mice receive a DNA boost to mobilize the memory T cells into activated effector cells. 7-12 days later, the activity of mobilized effector cells is quantified by determining their ability to specifically kill HBsAg peptide-pulsed targets that are adoptively transferred intravenously into the mice. The mice receive two populations of cells from naïve mice that are differentially labeled with the intravital dye CFSE (fluorescence emission in the FITC channel): $CFSE^{low}$ cells that are not pulsed with peptide, and $CFSE^{high}$ cells that are pulsed with the L (d)-restricted immunodominant peptide from HBsAg (28-39 of HBsAg). The disappearance of the peptide-pulsed $CFSE^{high}$ population relative to the unpulsed $CFSE^{low}$ population is the measure of peptide-specific killing according to the formula shown below. The percentages of these two populations in the cohorts of naïve recipients serves as the internal control for cell engraftment. A summary of this assay is shown below.

**Target cell preparation:**

Naïve splenocytes: Label with $CFSE^{low}$. Pulse without peptide. Verify labeling by flow cytometry.

Naïve splenocytes: Label with $CFSE^{high}$. Pulse with peptide. Verify labeling by flow cytometry.

Mix equal numbers of $CFSE^{low}$ and $CFSE^{high}$ cells (1-1.5 x $10^7$ cells of each).

***In vivo* CTL assay:**

Transfer cells to cohorts of both immunized and naive mice intravenously.

20-24 hours later, harvest spleens and process for flow cytometry and ELISPOT assays.

Ratio = Percentage of $CFSE^{low}$ cells: $CFSE^{high}$ cells

$$\% \text{ Specific Lysis} = 1 - \frac{\text{ratio in naïve mice}}{\text{ratio in immunized mice}} \times 100$$

### Measurement of protective muscosal immunity following intranasal challenge with live recombinant vaccinia virus expressing HBsAg (VV-HBsAg)

Hepatitis B virus is highly species- specific and only infects humans and some non-humans primates. To circumvent this issue, recombinant virus expressing HBsAg was used for live virus challenge in mice to test the protective eficacy of the HBV vaccination protocols. We used a genetically engineerd attenuated vaccinia virus vector expressing the HBsAg antigen (VV-HBsAg) to challenge mice, kindly provided by Dr. Bernard Moss, Laboratory of Viral Diseases, NIAID, NIH (Smith, Mackett and Moss. 1983. Nature 302:490-495). The feasibility and utility of using recombinant vaccinia virus expressing pathogen antigens for virus challenge has been demonstrated recently in mice in several virus disease models, including hepatits C and HIV (Pancholi, P. et al. 2004. J. Virol. 77(1):382-90; Marata, K. et al. 2003. Proc. Natl. Acad. Sci. USA 100(11); Belyakov, IM et al. 1998. Proc. Natl. Acad. Sci USA 95(4): 1709-14).

Naïve or immunized mice were infected intranasally with 2 x $10^5$ PFU of virus in a 50 μl volume. Mice were sacrificed after 5 days, and viral titers were determined in lung homogenates. Lung washes and spleen cells were also evaluated for the presence of CTLs, defined by a $CD8^+$ $IFN\gamma^+$ phenotype.

### F. Detection of CTL by $CD8^+$ $IFN\gamma^+$ phenotype following intranasal challenge with live VV-HBsAg

CTLs can be defined by a surface $CD8^+$ intracellular $IFN\gamma^+$ phenotype according to well recognized and standardized protocols (for example, see reagents and kits offered by BD Biosciences, San Diego, CA). Using these reagents, the frequency of CTLs defined by phenotype was determined by flow cytometric analysis. For this purpose, mononuclear cells recovered from lung and spleen were activated with the HBsAg CTL peptide (28-39 of HBsAg) for five hours *in vitro,* in the presence of a golgi inhibitor to prevent secretion of accumulated intracellular cytokines. Cells were then surface stained for CD8, permeabilized, and stained for intracellular IFNγ according to the manufacturer's directions.

VIII. Examples

This invention is further illustrated by the following examples which should not be construed as limiting.

In the following examples, the hepatitis B virus surface antigen (HBsAg) was chosen as an illustrative antigen for the subject liposome-based vaccines. Hepatitis B infection is a significant health risk and is one of the leading causes of death worldwide. Antibody response against HBsAg is known to confer protection against live virus infection in 5-10% of humans. HBsAg is a well characterized antigen that contains numerous T and B cell epitopes making it a suitable model antigen to demonstrate immune responses in mice. In addition, recombinant hepatitis B virus protein and gene expression constructs are also commercially available, eliminating the requirement for costly reagent development. However, it should not be construed that the invention is limited to the HBsAg vaccine. Rather, the instant invention has broad uses in a wide-range of pathogens, including various viruses and bacteria.

**EXAMPLE 1: Effect of Prime and Boost Immunization Protocol on Antibody Responses**

To immunize a host animal such as CD1 or Balb/c mice, primary immunization using the subject HBsAg-liposome preparation was administered to the animal on week 0. At week 6, HBsAg-liposome secondary boost immunization was administered.

To optimize the vaccine delivery platform, we performed several experiments using different immunization strategies, as outlined in Examples 1-5. In this series of experiments, liposomes were sized at 4 μm, a size reported to be effective in stimulating immune responses to protein antigens. Initial experiments tested the following parameters: the effect of one (prime week 0) versus two (prime and boost weeks 0 and 6) rounds of immunization (Example 1), kinetics of the antibody response (Example 2), dose of HBsAg-liposome preparations (Example 3; 3 μg or 15 μg/mouse), route of administration (Example 4; intranasal or intramuscular), and physical form of the vaccine (Example 5; fresh or lyophilized). Samples were collected at the indicated time points for analysis of humoral and mucosal immune responses.

Negative controls included naïve mice, liposomes alone, and HBsAg protein alone. Animals were also immunized with either 3 μg GSK vaccine intramuscularly (Engerix™-B from GSK Biologicals, or "GSK HBsAg,") as positive control. Both outbred, genetically heterogenous CD1 mice (N = 225 at 5/group) and inbred Th2-prone Balb/c mice (N = 50 at 5/group) were immunized for comparison purposes. CD1 mice are an outbred strain (i.e., genetically heterogeneous) and are therefore representative of the diverse genetics found in humans.

For example, in one series of experiments, 10 mice were immunized at week 0 by prime antigen. At week 6, a boost dose was administered to 5 of the 10 mice. Samples were collected (for example, from blood, feces, and vaginal wash) at weeks 2, 4, 6, 8, and 12 weeks for antibody determinations by ELISA assays..

Examples below show representative data from these studies to demonstrate the optimal conditions for vaccination with the subject HBsAg-liposomes. The humoral immune response to the different vaccines was measured by determining HBsAg-specific total serum antibody responses using ELISA assays that we developed (*supra*). Although samples taken at 2, 4, 6, 8, and 12 weeks after priming were analyzed, only the responses at eight weeks after the first immunization are shown for most examples.

Most graphs include responses to the commercial GSK HBsAg vaccine as a positive control, and to serve as a benchmark that we wish to achieve for the test vaccines. No HBsAg-specific responses were detected in any naïve mice (data not shown). Closed symbols identify responses from mice that received one immunization, while cross-hatched, closed symbols (+) show responses from mice that received both a priming and a booster immunization. Results are presented as the dilution of sample tested (x-axis) versus the optical density at 450 nm in ELISA assays for HBsAg-specific total serum antibody (y-axis). The ELISA results expressed as arbitrary units were read at 450 nm ($OD_{450}$).

For clarity, most of the data presented here are averages from samples pooled from 5-10 mice per experiment (equal amounts of individual samples from groups of mice). Figure 2 is representative of the degree of variation that is observed between individual CD1 and Balb/c mice within an experiment.

In Figures 2-8, it is shown that for all conditions examined with the exception of a 3 μg dose of HBsAg-liposome delivered intranasally (Figure 4), a booster immunization substantially enhanced HBsAg-specific serum antibody responses to both HBsAg-liposomes and to the GSK HBsAg vaccine control. Liposomes are therefore a very effective adjuvant and delivery vehicle for the HBsAg antigen and antibody responses are increased substantially after an identical second booster immunization.

**EXAMPLE 2: Kinetics of Antibody Responses in CD1 Mice after Intranasal (IN) Immunization with HBsAg Encapsulated in Liposomes (HBsAg-liposomes)**

Figure 3 illustrates the serum titer of HBsAg-specific antibodies after immunizing CD1 mice intranasally with 15 μg of HBsAg-liposome. A significant boost in titer of HBsAg-specific antibody was observed if a second boost administration of the same Ag preparation was used. Without boost administration, however, titer reached its peak at about 4 weeks post the initial immunization, and stayed at similar levels through week 8. In addition, 15 μg of HBsAg-liposome is at least as effective as the commercially available GSK vaccine at 3 μg dosage.

### EXAMPLE 3: Dose Response to HBsAg-liposomes

CD1 mice were immunized, with or without a second boost administration, with either 3 μg or 15 μg of HBsAg encapsulated in the liposome preparation. Figure 4 shows that 15 μg of HBsAg-liposome was at least as effective as the commercially available GSK vaccine (3 μg). However, 3 μg of HBsAg in the same liposome preparation was significantly less effective, and a second boost administration did not appear to result in a significant increase in titer.

### EXAMPLE 4: Routes of Administration of HBsAg-liposomes

To test the effect of administration routes, CD1 mice and Balb/c mice were immunized with the same HBsAg-liposome preparation as described above, through intranasal (IN) or intramuscular (IM) administration. Antibody titers were measured 8 weeks post (the initial) immunization.In both CD1 and Balb/c, both IN and IM administration of 15 μg of HBsAg-liposome, with or without a second boost, were at least as effective as 3 μg of the GSK vaccine (Figures 5 and 6.

### EXAMPLE 5: Effects of Fresh or Lyophilized HBsAg-liposome Preparations on Antibody Responses

To test whether the HBsAg-liposome preparation has to be used as fresh or lyophilized form, CD1 mice were immunized with either fresh or lyophilized liposome preparations, either by intranasal or intramuscular routes of administration. All serum Ig titers were measured 8 weeks post initial immunization.

Intranasal administration of HBsAg-liposome was at least as effective as the 3 μg GSK vaccine control if the liposome preparation was fresh (Figure 7). Comparable levels of titers were observed between the GSK vaccine and the fresh HBsAg-liposome preparation, either with or without boost. Lyophilized HBsAg-liposomes were slightly less effective than the fresh preparation in this experiment. In another separate experiment, however, lyophilized HBsAg-liposome preparation was slightly better that the fresh counterpart (data not shown).

Similar result was obtained with IM administration (Figure 8). Both fresh and lyophilized HBsAg-liposome preparations had similar final titers as compared to each other, and to the GSK positive control.

These results indicate that reconstituted preparations of lyophilized HBsAg-liposomes were generally as effective as the fresh preparations when delivered by the IN or the IM routes. The efficacy of the lyophilized preparations will simplify the distribution and storage of future vaccine formulations.

### EXAMPLE 6: The Effect of Liposome Size on Antibody Responses to HBsAg-liposomes

The effect of liposome size was evaluated by encapsulating HBsAg (15 μg/mouse) in liposomes sized at 4 μm, 1 μm and 0.2 μm. Quantitative ELISA assays were used to measure HBsAg-specific serum IgG levels after intranasal immunization with single sized liposomes or after intranasal immunization with an equal mixture of all three sizes. It is possible that a mixture of different sized liposomes may be more immunogenic than a single size of liposome. Total amounts ofHBsAg and lipid were the same in the four groups.

Figure 9 shows that liposomes sized at 1 μm and 4 μm were most effective, while the smaller 0.2 μm liposomes were less effective at generating HBsAg-specific serum IgG. A mixture of equal parts of all three sizes generated an additive but not a synergistic response. The predicted response if the effects of the three sizes of liposomes combined is additive is shown on Figure 9 as a vertical bar.

### EXAMPLE 7: The Effect of Liposome to Protein Ratio on Antibody Responses to HBsAg-liposomes

We determined the importance of liposome to protein ratios in generating HBsAg-specific serum IgG antibodies by immunizing mice intranasally with HBsAg-liposomes (15 μg/mouse, primed at week 0 and boosted at week 6) at our standard content of liposomes, or at 1/2 and 1/3 of the liposome concentration. Reducing the liposome to HBsAg ratio by 1/2 and 1/3 reduced serum IgG responses disproportionately to 35% and 2% of the response obtained from our standard liposome preparation, respectively (n = 8 animals per group; serum IgG measured on pools of sera from each group two weeks post-boost). The amount of liposome used to encapsulate the HBsAg therefore has a significant effect for generating high levels of HBsAg-specific serum antibody responses.

### EXAMPLE 8: Characterization of Polarized Th Responses Following Homologous Immunization Protocols

Cytokines are the hormonal messengers responsible for most of the biological effects in the immune system, such as cell mediated immunity and allergic type responses. Although they are numerous, cytokines can be functionally divided into two groups: those that are pro-inflammatory and those that are essentially anti-inflammatory but that promote allergic responses.

T lymphocytes are a major source of cytokines. These cells bear antigen specific receptors on their cell surface to allow recognition of foreign pathogens. They can also recognize normal tissue during episodes of autoimmune diseases. There are two main subsets of T lymphocytes, distinguished by the presence of cell surface molecules known as CD4 and CD8. T lymphocytes expressing CD4 are also known as helper T cells, and these are regarded as being the most prolific cytokine producers. This subset can be further subdivided into Th1 and Th2, and the cytokines they produce are known as Th1-type cytokines and Th2-type cytokines.

Th1-type cytokines tend to produce the pro-inflammatory responses responsible for killing intracellular parasites and for perpetuating autoimmune responses. Interferon gamma (IFN-γ) and interleukin-12 (IL-12) are the main Th1 cytokines. CTL activity is also high in a Th1-type immune response. In contrast, the Th2-type cytokines include interleukins 4,5, and 13, which are associated with the promotion of IgE and eosinophilic responses in atopy, and also interleukin-10 (IL-10), which has more of an anti-inflammatory response. Th2 response provides help for the maturation of B cells to immunoglobulin-secreting cells, thereby primarily activating humoral defense mechanisms. CTL activity is generally low in a Th2-type immune response. In excess, Th2 responses will also counteract the Th1 mediated microbicidal action.

In general, the optimal scenario seems to be that humans should produce a well balanced Th1 and Th2 response, suited to the immune challenge. However, in reality, central to the concept of Th1 and Th2 subset generation is the tendency for these responses to become polarized. Thus, a Th1 or Th2 cytokine-producing profile will often dominate during an immune response by preferentially amplifying one Th subset and down-regulating the opposing response. This polarized response appears to be critical for host defense against many pathogenic organisms. Resistance to intracellular pathogens (such as virus) often requires a predominantly Th1 response, while Th2 responses are typically needed to fight extracellular parasites. Thus, T cell-derived cytokines produced by the host in response to an infectious agent determine the outcome of infection in many infectious disease models.

Whereas a pro-inflammatory Th1 response is usually required to control intracellular infections, there is also a need to balance the response (Taylor-Robinson, Int J Parasitol. 28(1): 135-48, 1998). It is important to produce a sufficiently potent type 1 response to keep the intracellular infection under control, while producing at the same time just enough of a type 2 or immunosuppressive response to prevent the protective response from causing damage to the host. The available data suggest that IFN-γ, IL-12, and IL-10 cooperate to keep the Th2 response in check. Therefore, a successful outcome following an infection requires precise titration of Th1 and Th2 responses, appropriate to the type of infection. This is not just in terms of amount but also where, when and for how long these responses occur.

Several factors have been proposed, including the properties of antigens, dose of antigen, site of exposure and ongoing immune response in the host, to push a T-cell response towards a predominantly Th1 or Th2 phenotype. T helper cell responses to antigens may be characterized as polarized or as mixed. Polarized Th cell responses result from a skewing of the antigen-specific Th cell population towards a Th1 or a Th2 cytokine profile, and are reflected by antigen-specific IgG1:IgG2a ratios of <0.5 and >2.0, respectively. Mixed Th cell responses may contain both populations of T cells, and are reflected by antigen-specific IgG1:IgG2a ratios between 0.5 and 2.0. See Table B below.

Table B Characteristics of Polarized Th1 and Th2 Responses

| Immune Response | Type 1 (Th1) | Type 2 (Th2) |
|---|---|---|
| Humoral Immunity | IgG1:IgG2a ratio < 0.5 | IgGI:IgG2a ratio > 2.0 |
| Cytokine Secretion | ↑ IFN-γ, IL-12;<br>↓ IL-4, IL-10 | ↑ IL-4, IL-10;<br>↓ IFN-γ |
| CTL Activity | High | Low |

The nature of the immune responses to HBsAg-vaccine formulations was evaluated by determining IgG1:IgG2a ratios in the serum of CD1 and Balb/c mice at 8 weeks after the primary immunization. We designed quantitative ELISA assays to measure IgG1 and IgG2a antibodies specific for HBsAg. Figures 10 and 11 show the IgG1:IgG2a ratios in the sera of CD1 (Figure 10) and Balb/c (Figure 11) mice immunized by commercial GSK HBsAg vaccine, HBsAg-DNA vaccine, and HBsAg-liposome vaccine delivered intranasally or intramuscularly. Two vertical lines at 0.5 and at 2.0 in each panel demarcate three different patterns of antibody response (Th1, mixed or neutral, or Th2) in the regions from left to right.

In agreement with published results, the commercial HBsAg vaccine preferentially generated a Th2-type immune response in both the outbred CD1 and the inbred Balb/c strains of mice. As expected, the HBsAg-DNA vaccine generated a Th1-type immune response in both strains. Administration of HBsAg-liposomes intranasally generated a mixed response in CD 1 mice and a Th2-biased response in Balb/c mice. In contrast, administration of HBsAg-liposomes intramuscularly generated a neutral immune response in both strains of mice.

GSK vaccine was the alum-based human formulation designed for intramuscular delivery (Engerix™-B from GSK Biologicals, adw subtype). HBsAg-DNA vaccine was a plasmid DNA (pRc/CMV-HBs(S) and was purchased from a commercial source (Aldevron, Fargo, ND). The plasmid expresses the small hepatitis B surface antigen under the control of the immediate-early CMV promoter. (Reference: Davis, H.L., M. L. Michel and R. G. Whalen. DNA-based immunization for Hepatitis B induces continuous secretion of antigen and high levels of circulating antibody. Human Molecular Genetics 2: 1847-1851. 1993).

**EXAMPLE 9: Unique IgA Responses Following Intranasal Delivery of HBsAg-liposomes**

Starting in the late 1960s, mucosal immunity was recognized as being an important defense against respiratory viruses when it was shown to correlate with protection against respiratory viruses in humans. At about the same time, the IgA class of antibodies was found to be especially prevalent in the respiratory tract and on other mucosal surfaces and to be the mediator of mucosal immunity. Other studies have since confirmed that mucosal immunity could ideally be stimulated in developing protection against infection through local vaccination.

Both IgG and IgA antibodies appear to be quite strain specific. With natural infection by respiratory viruses such as influenza virus, both a humoral and a systemic cellular immune response occur. The level of anti-viral antibody in the serum also correlates with protection.

One aspect of the instant invention provides a method to elicit mucosal immune responses by delivering HBsAg in liposomes directly to mucosal sites. An IgA-specific sandwich ELISA assay was designed to measure HBsAg-specific IgA in the serum and from mucosal sites. Serum, feces and vaginal washes from all of the experimental groups discussed above and some saliva samples were tested for IgA. End-point titers were established for each sample pool (N = 4-5 mice per pool) by serially diluting test samples. End-point titers were defined as an OD at 450nm in the IgA ELISA assay of greater than twice the value observed in samples from naïve non-immunized strain-matched mice at the same dilution of sample.

The only samples which showed detectable IgA responses were from mice that were given HBsAg-liposome vaccine by the intranasal route. Table C summarizes the results from the samples which scored positive in the HBsAg-specific IgA ELISA. IgA responses were detected in the serum and in most samples from mucosal sites after only one immunization. Boosting with a secondary immunization further increased IgA levels in most samples. Additionally, local immunization by the intranasal route generated secretory IgA responses at all remote mucosal sites sampled, including fecal pellets, vaginal washes, and saliva (data not shown for saliva due to the limited number of samples). These results demonstrate that immunization with HBsAg-liposomes by one route (intranasal) generated HBsAg specific mucosal IgA immunity at all of the mucosal sites sampled. The HBsAg-liposome vaccine, in contrast to the HBsAg protein or the liposomes alone, boosts serum immune Ig titers and also induces both serum and secretory IgA responses when administered intranasally.

All other immunogens fail to produce any detectable IgA responses (results not shown).

Table C. Summary of IgA responses in mice immunized with HBsAg-liposomes

| Immunization | Strain | Serum IgA | Fecal IgA | Vaginal IgA | Saliva IgA |
|---|---|---|---|---|---|
| HBsAg-liposome IN (1×) | CD1 | Medium | Negative | ND§ | NA¶ |
| HBsAg-liposome IN (2×) | CD1 | Medium | Low | ND§ | High |
| HBsAg-liposome IN (1×) | Balb/c | Low | Low | Medium | NA¶ |
| HBsAg-liposome IN (2×) | Balb/c | High | High | High | High |
| Samples of serum, feces, and vaginal washes were collected from five individual mice per group. Samples were processed individually, and sample pools were made using equivalent volumes of sample per mouse. End-point titrations were determined for each sample and were defined as a serum titer >2× that observed for naïve unimmunized CD1 or Balb/c mice. The range of titer values that were used for the determination of the relative titer are shown below.<br>§Not determined.<br>¶Sample not available. | | | | | |

| Relative titer | Serum | Feces | Vaginal Wash |
|---|---|---|---|
| Negative | <100 | <100 | <25 |
| Low | 100-200 | 100-200 | 25-100 |
| Medium | 400-800 | 400-800 | 200-400 |
| High | >1600 | >1600 | 400-1600 |

**EXAMPLE 10: Antibody responses to HBsAg-DNA Immunization Alone are Weak**

DNA vaccine immunization has recently been described to generate a protective immune response in a host, including humans. See U.S. Pat. No. 6,632,663 and WO 03/075955 A1 (incorporated herein by reference).

To test the effectiveness ofHBsAg-DNA as immunogen, CD1 mice and Balb/c mice were immunized intramuscularly with HBsAg-DNA, either with or without a second boost. Total HBsAg-specific serum Ig was measured 8 weeks post immunization as described before. GSK HBsAg vaccine was used as a positive control.

Figure 12 indicates that, in contrast to HBsAg protein vaccines with adjuvants, intramuscular immunization with HBsAg-DNA produced a weak antibody response to HBsAg in both CD1 and Balb/c strains of mice. The elicited immune response, even with a boost, is weak to moderate at best when compared to the unboosted control GSK vaccine.

**EXAMPLE 11: Homologous HBsAg-DNA Immunization Generates Potent CTL Activity and a Th1-Polarized Cytokine Profile**

HBsAg-DNA immunization is a weak inducer of antibody responses (Example 10), but is a very strong inducer of T cell responses. After immunization with HBsAg-DNA intramuscularly (100 μg on weeks 0 and 6, with a booster dose on week 11 to mobilize memory CTLs), an average of 87% ± 8% HBsAg-specific killing was observed using the *in vivo* CTL assay as a readout (n=4 mice). HBsAg-DNA immunization also strongly polarized the cytokine response by HBsAg-specific T cells towards a Type 1 cytokine profile. Figure 13 shows results from ELISPOT assays for IFN-γ and IL-10 by spleen cells from HBsAg-DNA immunized mice. Both HBsAg protein, which contains T cell epitopes, and the immunodominant CTL peptide from HBsAg (Ishikawa, T. et al. 1998. J. Immunol. 161:5842) generated high frequencies of IFN-γ secreting cells, but very low numbers of IL-10 secreting cells. Homologous immunization with HBsAg-DNA therefore generates a typical strongly polarized Th1-type profile, in agreement with the Th1-type IgG1: IgG2a ratios that were observed in two different strains of mice (Figures 10 and 11).

**EXAMPLE 12: Effects of Heterologous Immunization with HBsAg-DNA as Primer and HBsAg-liposome as Booster on Systemic and Mucosal Antibody Responses**

Although intramuscular DNA immunization with HBsAg-liposomes was a weak stimulus for the production of total antibody (Example 11), it was very effective at stimulating Th1-polarized cell mediated immune responses (Example 11). In order to determine whether DNA immunization was effective at generating memory T and B cell responses, we developed a heterologous immunization protocol. In this protocol, HBsAg-DNA immunization is used as the priming dose, and HBsAg-liposomes is used as the booster dose. If DNA is effective at generating T and B cell memory responses, then these may be revealed following a boost with a different form of the antigen (HBsAg-liposomes). Thus the invention provides a heterologous immunization experimental design. Variations of the protocol are merely routine and are well within the scope of invention (see Example 16 for permutations on the protocol).

In a representative protocol, mice were first immunized and primed with HBsAg-DNA vaccine intramuscularly, and then challenged with a low dose of HBsAg-liposome intranasally. In certain embodiments, the low dose is itself insufficient in eliciting a significant immune response when administered alone. When compared to a dose sufficient to elicit a full-scale immune response, the low dose is at most 50%, 40%, 30%, 20%, 10%, 5% or lower.

Balb/c mice were intramuscularly immunized by 100 μg of HBsAg-DNA at week 0, and again at week 6. At week 16, a boost of HBsAg-liposome was administered intramucosally at a reduced dose of 3 μg. Serum samples were obtained 2, 4, and 6 weeks following the boost. As a positive control, CD1 mice were immunized intranasally with 15 μg of HBsAg-liposome with a second boost. Positive control serum was obtained 8 weeks post initial immunization. As a negative control, sera from naïve CD 1 mice were obtained at week 8.

Figure 14 shows results from this heterologous immunization protocol. Two rounds of IM HBsAg-DNA immunizations or one round of IN HBsAg-liposome (low dose) immunization alone induced weak serum antibody responses. However adding a second low dose boost with HBsAg-liposomes (3 μg) IN to the DNA immunization, regimen markedly increased the total HBsAg-specific antibody response to a level even higher than seen in mice following two rounds of IN HBsAg-liposomes (high dose).

Quantitative ELISA assays were used to determine microgram amounts of HBsAg-specific IgG in the sera from this experiment. IgG levels after two rounds of DNA averaged 43.2 μg/ml, and increased to 248 μg/ml after boosting with HBsAg-liposomes. Administration of the liposome preparation alone generated only 0.6 μg/ml of serum IgG.

Remarkably, DNA immunization also primes the immune system for mucosal IgA responses, which also are mobilized following intranasal delivery of a low dose HBsAg-liposome boost. Figure 15 shows that the DNA and liposome components of the heterologous immunization regimen alone fail to stimulate detectable IgA responses. However substantial IgA responses were induced in the serum and in the vagina using the heterologous DNA prime and HBsAg-liposome boost protocol. Secretory IgA was also induced in fecal samples using this protocol (data not shown). Figure 15 also shows typical serum IgA responses following prime and boost with a higher dose of HBsAg-liposomes alone (15 μg HBsAg), which are a benchmark for robust IgA responses. The boost with HBsAg-liposomes must be delivered by the intranasal route in order to generate serum or mucosal IgA responses. If the boost is given IM rather than IN, only low level of secretory IgA is generated although circulating serum IgG responses are robust (data not shown).

IgG antibodies specific for HBsAg were also detected in the lung and vaginal washes from mice administered the heterologous immunization protocol (Figure 16). Neither untreated mice nor mice that received HBsAg-DNA IM followed by blank liposomes IN, generated detectable IgG levels in the lung or vaginal washes that were above background values. However mice that were immunized with a combination of HBsAg-DNA IM followed by HBsAg-liposomes IN produced readily detectable mucosal IgG at both the lung and vaginal sites. This is relevant since mucosal IgG has also been shown to play a protective role in protecting against certain pathogens such as HSV-2 and influenza (Parr, E.L. et al. 1997. J. Virology 71:8109-8115 for HSV-2; Tamura, S. and T. Kurata. 2004. Jpn. J. Infect. Dis. 57(6):236-47; Renegar, K.B. et al. 2004. J. Inmunol. 173(3):1978-86).

Applicants also determined the IgG1:IgG2a ratio to evaluate the effect of the IN boost with HBsAg-liposomes on polarization of the T cell response following the heterologous immunization protocol. Figure 17 shows the ratios of IgG1: IgG2a in the sera of individual mice after the HBsAg-liposome boost. Significantly, adding this boost to the DNA immunization protocol did not shift immune responses towards a Th2-type profile. Antibody responses 4 weeks after the second DNA immunization showed a Th1-type bias (IgG1: IgG2a ratio of 0.47). A Th1-biased response was still present after an intranasal boost with HBsAg-liposomes (IgG1: IgG2a ratio of 0.50). This experiment shows that DNA immunization establishes a strong and stable base of Th1-type memory which is effectively mobilized when HBsAg-liposomes are delivered intranasally.

The subject heterologous immunization protocols promote neutral or type 1 polarized Th responses and cell mediated immunity, while optionally inducing mucosal IgA and IgG responses (if the boost dose antigen-liposome preparation is administered intranasally). For protective immunity from intracellular pathogens, it is often desirable to initiate cell mediated immune responses, particularly for enhanced Th1 cytokine secretion and for the generation of CTLs. This is particularly useful for certain embodiments, where it may be desirable that an antigen and/or immunization protocol results in a Th1 response, rather than a Th2 or mixed-type response, since Th2 responses may be associated with certain undesirable side-effects similar to allergic reaction, especially in children.

In other embodiments, where the initial dose is the subject HBsAg-liposome preparation administered intranasally, and the boost dose is HBsAg-DNA administered intramuscularly, the resulting moderate immune response in the host animal tend to be a mixed-type, rather than a Th1 type response. This illustrates the contention that the order of the subject heterologous immunization is important in order to achieve the Th1 response.

## EXAMPLE 13: Effects of Heterologous Immunization with HBsAg-DNA as Primer and HBsAg-liposome as Booster on the Avidity of Serum IgG Antibodies to HBsAg

Antibody avidity (ie, the overall strength of antigen-antibody binding) can play an important role in the control of pathogens, with higher avidity antibodies generally more effective than low avidity antibodies. To determine the binding strength of HBsAg-specific IgG serum antibodies for the HBsAg antigen, a quantitative IgG avidity assay was developed using the chaotropic reagent urea to disrupt antigen-antibody complexes. Higher avidity antibodies require higher concentrations of urea to disassociate the antigen bound to the antibodies. Figure 18 shows representative results, of the IgG avidities in mice immunized by a homologous immunization regimen (left panel) and a heterologous immunization regimen (right panel). Results are represented as avidity indices, which are μg of free IgG in the absence of urea divided by μg of free IgG in the presence of urea. The maximum ratio that can be obtained in this assay is 1.0, which is shown as a dotted line at the top of each graph.

Homologous immunization with HBsAg-liposomes generates avidities that closely parallel those obtained with the current GSK HBsAg alum-based vaccine used in humans. Immunization with HBsAg-DNA also generates good avidity responses. Heterologous immunization with HBsAg-DNA followed by HBsAg-liposomes increases the antibody avidities even further, surpassing the reference response of the commercial human vaccine. Delivery of the HBsAg-liposome boost intranasally results in the highest avidity antibodies; however IM delivery is also very effective. Higher avidity antibodies are predicted to be more effective at antigen neutralization, illustrating the superiority of the heterologous immunization protocol over homologous immunization regimens using the commercial GSK vaccine (IM) or HBsAg-liposomes (IN or IM).

**EXAMPLE 14: Heterologous Immunization with HBsAg-DNA as Primer and HBsAg-liposome as Booster Overcomes Non-Responsiveness to HBsAg in C57BL/6 Mice**

C57BL/6 mice are non-responsive to the HBsAg protein (ref Schirmbeck, R. et al. J. Virol. 69(10):5929-34), as are approximately 5-10% of humans who receive the commercial GSK vaccine for HBsAg. To determine whether heterologous immunization can induce immune responses in non-responder mice, the following experiment was performed (Table D). One group (Group A) of C67BL/6 mice was immunized with two rounds of HBsAg-liposome delivered intranasally. As expected, these mice failed to generate a detectable level of anti-HBsAg serum IgG. These mice and a second group of naïve mice (Group B) were then immunized with two rounds of HBsAg-DNA delivered intramuscularly. Serum IgG levels in both groups of mice in response to DNA vaccination were comparable (12.8 μg/ml and 8.0 μg/ml, for Groups A and B, respectively). Each group was then further boosted with HBsAg-liposome prior to determination of final serum IgG levels. Surprisingly, both groups of C57BL/6 mice demonstrated equivalent high levels of anti-HBsAg serum IgG (Group A, 238μg/ml; Group B, 310μg/ml). The results from this experiment illustrate three points: 1) heterologous immunization can stimulate high antibody responses in non-responsive mouse strains; 2) the order of immunization is critical for the induction of serum IgG responses; 3) synergistic antibody responses are observed after heterologous immunization.

**Table D. HBsAg-DNA immunization followed by HBsAg-liposomes IN overcomes non-responsiveness to HBsAg in C57BL/6 mice**

| Treatment Groups | A: Serum IgG(μg/ml) | B:Serum IgG (μg/ml) |
|---|---|---|
| A. 2x HBsAg-liposomes IN | 0 | --- |
| B. 2x HBsAg-DNA IM | 12.8 | 8.0 |
| C. HBsAg-liposomes IN | 238 | 310 |

**EXAMPLE 15: Heterologous Immunization Generates *in vivo* CTL Activity and Th1-biased Cytokine Secretion**

Protective immunity from many intracellular pathogens often requires a Th1-biased immune response with high IFN-γ production, and the generation of cytolytic T lymphocytes (CTLs) which can kill pathogen infected cells. To evaluate the cell mediated immune responses generated by our vaccine delivery protocols, Applicants have established *in vivo* assays to measure CTL responses, and ELISPOT assays to enumerate frequencies of HBsAg-specific IFN-γ and IL-10 secreting cells (Th1 and Th2 responses, respectively).

*In vivo* CTL assays have been recently reported in the literature. Unlike conventional *in vitro* CTL assays, they provide a true measure of the ability of CTLs to kill targets in the *in vivo* setting. Applicants measured *in vivo* CTL activity in mice that received two rounds of HBsAg-DNA IM followed by a low dose IN HBsAg-liposome boost. After the sample collection was completed for the measurement of antibody responses (six weeks or longer after the IN boost), mice received a DNA boost to mobilize the memory T cells into activated effector cells. 7 days later, the activity of mobilized CTL effector cells was quantified by determining their ability to specifically kill HBsAg peptide-pulsed dye labeled targets that were adoptively transferred intravenously into the mice, as outlined in Methods. The disappearance of the peptide-pulsed dye-labeled $CFSE^{high}$ population relative to the unpulsed dye-labeled $CFSE^{low}$ population is the measure of peptide-specific killing. The percentages of these two populations in cohorts of naive recipients served as the internal control for cell engraftment. The percentage of $CFSE^{low}$ and $CFSE^{high}$ donor cells in the spleens of the recipients was determined by flow cytometric analyses

Representative flow cytometric analyses are shown in Figure 19 for one assay. The left panel shows $CFSE^{low}$ (R2 on the graph) and $CFSE^{high}$ (R3 on the graph) populations of donor cells that were recovered from the spleens of naive mice 22 hours after injection of the CFSE labeled target cells. CFSE fluorescence is shown in the FITC or FL1 channel on the x-axis. FL1 fluorescence is displayed versus irrelevant FL2 fluorescence solely for the purpose of excluding auto-fluorescing cells from the analysis. The right panel shows the same analysis from mice that had received the heterologous immunization protocol. The disappearance of $CFSE^{high}$ (specific peptide pulsed) population from the R3 region indicates specific killing by CTLs.

High levels of HBsAg peptide-specific CTL killing activity were observed in Balb/c mice that were primed intramuscularly with HBsAg-DNA and boosted intranasally with HBsAg-liposomes. *In vivo* CTL activity averaged 78% (range of 65-91 % specific killing) in a grouping of four mice.

Spleen cells from mice that received the heterologous immunization regimen were also cultured *in vitro* for the measurement of IFN-γ and IL-4 secreting cells by ELISPOT assays. The ELISPOT assays were established using commercial antibody pairs and reagents (BD Pharmingen) and Multiscreen-IP multiwell ELISPOT plates (Millipore, Hopkington, MA). *In vitro* cultures were stimulated with HBsAg peptide at a concentration of 10 μg per 2.5 $\times 10^6$ cells per ml for 20-22 hours. Cultures with no peptide were used as a negative control, and cultures stimulated with anti-CD3 or Con A were used as positive controls. Spots were quantitatively analyzed by CTL Inc.

Applicants observed high frequencies of HBsAg-specific IFN-γ secreting cells, but only very low frequencies HBsAg-specific IL-10 secreting cells in mice immunized with the heterologous immunization protocol (data not shown). These results directly confirm our conclusion derived from the analysis of serum IgG1: IgG2a ratios, that the heterologous immunization protocol generates a Th1-type cytokine biased immune response.

**EXAMPLE 16: Variations in the Delivery Protocol for the HBsAg-DNA and HBsAg-liposome Components of the Heterologous Immunization Protocol**

Further studies determined the extent to which the two parameters of the heterologous immunization protocol, DNA priming and antigen-liposome boosting, could be varied while still retaining good immunogenicity for CTL and/or antibody responses. Table E shows the results of *in vivo* CTL killing activity and serum IgG responses in mice where the priming dose of DNA was varied with respect to dose and tempo of delivery. The first protocol (A) shown consists of DNA delivery on weeks 0 and 3 (total dose of 200 μg) and HBsAg-liposome boost on week 6. This protocol generates near maximal specific CTL killing activity and high levels of HBsAg-specific serum IgG, as has been shown in previous examples. Reducing the DNA from 2 administrations totaling 200 μg to one administration totaling 100 μg (B) produces the same levels of CTL and antibody responses. Further modification consisting of dividing the 1x DNA priming dose into two parts delivered within a three day time span, and shortening the liposome boost administration by three weeks (C) preserved the high CTL killing activity and antibody response. At a reduced DNA priming dose (D) there is an indication that both CTL and antibody levels are beginning to diminish. At 100-fold less DNA (E), CTL activity is still approximately 2/3 of maximum, whereas antibody levels are nearly undetectable. Thus significant variations in both dose and timing of the DNA booster immunization are possible while still preserving the desired outcome of high CTL and antibody levels.

**Table E. Effect of different DNA priming regimens in generating *in vivo* HBsAg-specific CTL killing activity and serum IgG in Balb/c mice**

| Treatment groups | % killing *in vivo* | Serum μg/m, |
|---|---|---|
| **A.** 2x DNA on wks 0, 3 (200 μg total) + HBsAg-lipo IN wk 6 | 87 ± 8 | 176 |
| **B.** 1x DNA in 3 days (100 μg total) + HBsAg-lipo IN week 6 | 91 ± 4 | 197 |
| **C.** 2x DNA in 3 days (100 μg total) + HBsAg-lipo IN week 3 | 90 ± 5 | 318 |
| **D.** 2x DNA in 3 days (10 μg total) + HBsAg-lipo IN week 3 | 76 ± 14 | 125 |
| **E.** 2x DNA in 3 days (1 μg total) + HBsAg-lipo IN week 3 | 61+22 | 0.5 |
| Legend to Table E. Groups of 5 mice were immunized as indicated. Serum pools were collected two weeks after the last immunization for HBsAg-specific serum IgG determinations. *In vivo* CTL assays were performed on individual mice 7 days after administration of a boost of 1x DNA (100 μg) IM as a stimulus to help reveal the differences in DNA priming. Numbers in parentheses under the treatment groups indicates the total DNA dose administered per mouse. | | |

The second variable examined was the volume of HBsAg-liposome delivered intranasally to mice that had been primed with HBsAg-DNA. This was achieved by mixing HBsAg and empty liposomes together, keeping the total amount of delivered protein at 15 μg /dose and the liposome content constant. This protocol was possible because it was shown that admixture ofHBsAg and liposomes gives responses that are approximately 70% of responses seen when HBsAg is encapsulated in liposomes. Serum anti-HBsAg specific IgG levels were found to be within a similar narrow range after instillation of either 50, 40 or 30 μl of inoculation volume.

**Table F. Effect of HBsAg-liposome volume on antibody responses using the heterologous immunization protocol**

| | *Week 0 Immunization* | *Week 3 Immunization* | *Week 5 Antibody Detennination* |
|---|---|---|---|
| Group¶ | 2 x 50 μg IM | Total volume for IN Immunization | Serum anti-HBsAg IgG μg /ml |
| A | HBsAgDNA | 50μl (15 μg HBsAg + liposome) | 198 |
| B | HBsAg-DNA | 40 μl (15 μg HBsAg + liposome) | 285 |
| C | HBsAg-DNA | 30 μl (15 μg HBsAg + liposome) | 309 |
| ¶N=8 mice per group. | | | |

**EXAMPLE 17: Heterologous Immunization (HBsAg-DNA Prime and HBsAg-liposome Boost) Generates Mucosal CTL and Provides Complete Protection Against Live Virus (VV-HBsAg) Challenge**

To determine whether the heterologous immunization regimen generated local mucosal or systemic CTL, defined by their $CD8^+$ $IFN\gamma^+$ phenotype, Balb/c mice were immunized with two rounds of HBsAg-DNA IM followed by one round of HBsAg-liposome IN. Balb/c mice immunized with two rounds of HBsAg-DNA followed by one round of empty liposomes, and non-immunized mice served as controls. The mice were challenged intranasally with recombinant vaccinia virus expressing the HBsAg (VV-HBsAg) 14 days after the liposome immunization. Lung (local mucosal compartment) and splenic (systemic compartment) mononuclear cells were isolated and tested for the presence of CTL five days after virus challenge. Antigen-specific CTL were identified by their $CD8^+$ $IFN\gamma^+$ phenotype determined by flow cytometric analyses. Table G shows that naïve mice generated few CTL in either the lung or the spleen. Substantial numbers of CTL were generated in the lung of mice immunized with DNA and empty liposomes, and much smaller numbers were also generated in the spleen. However mice immunized with a combination of DNA and HBsAg-liposomes generated a massive expansion of HBsAg-specific CTL locally in the lung, and significant numbers in the splenic compartment. These data demonstrate that both elements of this heterologous immunization protocol generate large numbers of antigen-specific local CTL in the lung mucosa following a challenge with virus. Significantly, antigen-specific CTL are also detected in the splenic compartment indicating a systemic response of antigen-specific CTL.

**Table G. Generation of $CD8^+$ $IFN\gamma^+$ CTL after heterologous immunization**

| Treatment groups§ | % $CD8^+$ $IFN\gamma^+$ in lung$^\zeta$ | % $CD8^+$ $IFN\gamma^+$ in spleen$^\zeta$ |
|---|---|---|
| Untreated | 0.9% | 0.05 ± 0.05% |
| HBsAg-DNA 2x Blank liposome 1x | 38% | 0.44 ± 0.11% |
| HBsAg-DNA 2x HBsAg-liposome 1x | 73% | 1.79 ± 0.31% |
| § Balb/c mice were immunized as indicated (treatment groups; n= 5 mice per group) at intervals of four weeks. Mice were immunized with 100 μg of HBsAg-DNA intramuscularly (50 μl per quadriceps), and 50 μl of either empty of HBsAg-liposomes intranasally. Mice were infected with $2 \times 10^5$ PFU of VV-HBsAg intranasally in a volume of 50 μl 14 days after the last immunization. ζ Immunofluorescence analysis was performed on lung mononuclear cells (n= 1 per group) and on splenocytes (n=5 per group) five days after virus challenge. Cells were stimulated *in vitro* for five hours with 10 μm of HBsAg CTL peptide per $10^6$ cells. Cells were then stained for surface CD8 and intracellular IFN-γ. | | |

Figure 20 shows results from a VV-HBsAg challenge delivered intranasally to three cohorts of mice: untreated mice, mice that received HBsAg-DNA and blank liposomes IN, and mice that received HBsAg-DNA and HBsAg-liposomes IN. Log PFU's (virus plaque forming units) per lung were determined five days after a challenge with VV-HBsAg. Untreated naïve mice have high titers of virus in the lung, averaging 3.72 log PFU's per lung (11,038 PFU's). Mice that received DNA and plain liposomes had two logs fewer PFU's per lung than untreated mice (1.86 log PFU's or 156 PFU's). Mice that received DNA and HBsAg-liposomes had no detectable PFU's of virus in the lung, indicating complete clearance of the virus from the site of mucosal challenge. Since the HBsAg protein in this construct will only be expressed intracellularly, clearance of the virus is likely due to the activity of CTLs in the lung compartment.

## IX. SUMMARY

Table H summarizes the characteristics of the immune responses that were generated with specific HBsAg vaccine delivery platforms. Eleven different immunization protocols (homologous and heterologous) were developed and the immune responses to these protocols were characterized. Immunization schedules and vaccine components are as indicated. All antibody responses were determined two weeks after the last booster immunization. It should be noted that protocols 8 and 9 are shortened versions of the heterologous immunization platform. Using HBsAg as a model antigen, Applicants have shown that immune responses can be directed towards specific outcomes (i.e., antibody response levels, T helper cytokine profiles (Th1 v. Th2), development of secretory IgA mucosal immunity, and elicitation of cytolytic T cells). These studies provide a rational basis that can be used to tailor immune responses to generate protective immunity against specific pathogens under specific circumstances to favor Th1 or Th2 type immune responses, or a mixed / more balanced response.

Table H. Summary of the characterization of immune responses to HBsAg vaccines

| ***Experimental Design*** | Immunization (week 0) | Immunization (week 3) | Immunization (week 6) | Antibody response | Antibody avidity | Th type | Mucosal antibody responses |
|---|---|---|---|---|---|---|---|
| ***Homologous immunization*** | | | | | | | |
| 1 | Liposome, IM | liposome, IM | --- | high | medium | Th2 | no or low |
| 2 | Liposome, IN | liposome, IN | --- | medium | medium | Th2 | good |
| 3 | DNA-IM | DNA-IM | --- | low | medium | Th1 | no or low |
| 4 | GSK HBsAg vaccine, IM | GSK HBsAg vaccine, IM | --- | high | medium | Th2 | no or low |
| ***Heterologous Immunization*** | | | | | | | |
| 5 | DNA, IM | DNA, IM | liposome, IN | high | high | Th1 | good |
| 6 | DNA, IM | DNA, IM | liposome, IM | high | high | Th1 | no or low |
| 7 | DNA, IM | DNA, IM | liposome+antigen mixture, IN | high | high | Th1 | good |
| | | | | | | | |
| 8 | DNA-IM | liposome-IN | --- | high | high | Th1 | good |
| 9 | DNA-IM, split dose in 3 days | liposome-IN | --- | high | high | Th1 | good |
| | | | | | | | |
| 10 | DNA-IM + liposome-IN | DNA-IM + liposome-IN | --- | high | ND | Th2 | good |
| | | | | | | | |
| 11 | Liposome, IN | liposome, IN | I DNA, IM | medium, no synergy | ND | Th2 | good |

Abbreviations: Liposome: HBsAg- encapsulated in liposome 1-4 μm in diameter
DNA: HBsAg-DNA
IM: intramuscular injection; IN: intranasal delivery

In summary, the results show that specific doses of HBsAg, when encapsulated in liposomes of a certain composition and size (such as 4 μm size average), are potent vaccines for the production of serum antibody. Robust responses are observed when the vaccine is administered intranasally or intramuscularly. In contrast, HBsAg-DNA injected intramuscularly is less effective in inducing HBsAg-specific serum antibody. Applicants did observe that the mice tolerated the intranasal vaccines in all formulations without evidence of distress or mortality.

Only intranasal immunization with HBsAg-liposome generated systemic and mucosal IgA responses, while all other immunization regimens failed to produce IgA response. And finally, heterologous immunization regimen with HBsAg-DNA vaccine by IM, followed by HBsAg-liposome by IN is an ideal vaccine delivery platform, in that it produces synergistic serum antibody response, robust mucosal and serum IgA response, and potentially enhanced antigen-specific T-cell response and IFN-gamma secretion without changing the Th1-type response established by DNA vaccine priming.

The biodistribution of HBsAg-liposomes was not measured directly. However the ability of vaccine formulations to stimulate local immunity at mucosal surfaces was determined and the results presented above.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Md. (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, Calif. (1990); Marshak et al., "Strategies for Protein Purification and Characterization-A Laboratory Course Manual" CSHL Press (1996); all of which are incorporated by reference as if fully set forth herein. Other general references are provided throughout this document. The procedures therein are believed to be well known in the art and are provided for the convenience of the reader.

## Claims

1. A priming preparation for intramuscular administration comprising a nucleic acid encoding an antigen: and a boosting preparation for intranasal administration comprising the antigen encapsulated in liposomes, for eliciting an immune response in an animal wherein administration of the boosting preparation produces an immune response comprising:

   (1) an increase in the serum level of antigen-specific IgA compared to the serum level of antigen-specific IgA prior to the administration of the boosting preparation and,
   (2) an increase compared to an untreated animal in the proportion of antigen specific CD8+IFNγ+ cytotoxic T lymphocytes in both the lung and the spleen.

2. The composition of claim 1 wherein the immune response elicited is biased towards a Th1 response in a host animal.

3. The composition of claim 1 or claim 2 wherein the desired immune response is against a pathogen and the antigen is a pathogen-specific antigen.

4. The composition of claim 1 wherein the liposomes in the boosting preparation average about 0.5-5 μm in size.

5. The composition of claim 3 wherein the immune response confers immunity against the pathogen.

6. The composition of claim 5 wherein the immune response protects animals from challenge by said pathogen.

7. The composition of one of claims 3-6 wherein the pathogen is a virus.

8. The composition of claim 7 wherein the virus is HBV, and the pathogen-specific antigen is HbsAg.

9. The composition of claim 8 wherein the animal is human.

10. The composition of claim 1, wherein the boosting preparation does not contain an adjuvant other than liposome.

11. A kit for eliciting an immune response in a host animal comprising:

a) a first immunising component for intramuscular administration comprising a nucleic acid encoding an antigen;
b) a second immunising component for intranasal administration comprising the antigen encapsulated in liposomes;
c) an instruction for a user to administer to the animal the first immunizing component followed by administering the second immunizing component to elicit an immune response in the animal.

12. The kit of claim 11 wherein the desired immune response is against a pathogen and the antigen is a pathogen-specific antigen.

13. The kit of claim 11 wherein the liposomes in the second immunizing component average about 0.5-5 μm in size.

14. The kit of claim 12 or claim 13 wherein the pathogen is a virus.

15. The kit of claim 14 wherein the virus is HBV, and the pathogen-specific antigen is HBsAg.

16. The kit of one of claims 11-15 wherein the animal is human.

17. The kit of claim 11, wherein the second immunizing component does not contain an adjuvant other than liposome.

18. The composition of claim 1, wherein the boosting preparation consists of antigen encapsulated in liposomes and a suitable solvent.

19. The kit of claim 11, wherein the second immunizing component consists of antigen encapsulated in liposomes and a suitable solvent.

## Patentansprüche

1. Prime-Präparat zur intramuskulären Verabreichung, das eine Nucleinsäure umfasst, die ein Antigen kodiert, und ein Boost-Präparat zur intranasalen Verabreichung, das das Antigen in Liposomen eingekapselt umfasst, zum Hervorrufen einer Immunreaktion in einem Tier, wobei die Verabreichung des Boost-Präparats eine Immunreaktion erzeugt, umfassend:

(1) eine Zunahme des Serumniveaus von antigenspezifischem IgA im Vergleich zum Serumniveau von antigenspezifischem IgA vor der Verabreichung des Boost-Präparats und,
(2) eine Zunahme des Anteils an antigenspezifischen CD8+IFNγ+zytotoxischen T-Lymphozyten sowohl in der Lunge als auch in der Milz im Vergleich zu einem unbehandelten Tier.

2. Zusammensetzung nach Anspruch 1, wobei die hervorgerufene Immunreaktion in Richtung einer Th1-Reaktion in einem Wirtstier tendiert.

3. Zusammensetzung nach Anspruch 1 oder Anspruch 2, wobei die gewünschte Immunreaktion gegen ein Pathogen gerichtet und das Antigen ein pathogenspezifisches Antigen ist.

4. Zusammensetzung nach Anspruch 1, wobei die Liposomen im Boost-Präparat eine durchschnittliche Größe von etwa 0,5-5 μm haben.

5. Zusammensetzung nach Anspruch 3, wobei die Immunreaktion Immunität gegen das Pathogen verleiht.

6. Zusammensetzung nach Anspruch 5, wobei die Immunreaktion Tiere vor einer Reizung durch das genannte Pathogen schützt.

7. Zusammensetzung nach einem der Ansprüche 3-6, wobei das Pathogen ein Virus ist.

8. Zusammensetzung nach Anspruch 7, wobei das Virus HBV ist und das pathogenspezifische Antigen HbsAg ist.

9. Zusammensetzung nach Anspruch 8, wobei das Tier ein Mensch ist.

10. Zusammensetzung nach Anspruch 1, wobei das Boost-Präparat kein anderes Adjuvans als Liposom enthält.

11. Kit zum Hervorrufen einer Immunreaktion in einem Wirtstier, der Folgendes umfasst:

a) eine erste Immunisierungskomponente zur intramuskulären Verabreichung, die eine Nucleinsäure umfasst, die ein Antigen kodiert;
b) eine zweite Immunisierungskomponente zur intranasalen Verabreichung, die das Antigen in Liposomen eingekapselt umfasst;
c) eine Anleitung für einen Benutzer zur Verabreichung der ersten Immunisierungskomponente an das Tier, gefolgt von der Verabreichung der zweiten Immunisierungskomponente, um eine Immunreaktion in dem Tier hervorzurufen.

12. Kit nach Anspruch 11, wobei die gewünschte Immunreaktion gegen ein Pathogen gerichtet ist und das Antigen ein pathogenspezifisches Antigen ist.

13. Kit nach Anspruch 11, wobei die Liposomen in der zweiten Immunisierungskomponente eine durchschnittliche Größe von etwa 0,5-5 μm haben.

14. Kit nach Anspruch 12 oder Anspruch 13, wobei das Pathogen ein Virus ist.

15. Kit nach Anspruch 14, wobei das Virus HBV ist und das pathogenspezifische Antigen HBsAg ist.

16. Kit nach einem der Ansprüche 11-15, wobei das Tier ein Mensch ist.

17. Kit nach Anspruch 11, wobei die zweite Immunisierungskomponente kein anderes Adjuvans als Liposom enthält.

18. Zusammensetzung nach Anspruch 1, wobei das Boost-Präparat aus in Liposomen eingekapseltem Antigen und einem geeigneten Lösungsmittel besteht.

19. Kit nach Anspruch 11, wobei die zweite Immunisierungskomponente aus in Liposomen eingekapseltem Antigen und einem geeigneten Lösungsmittel besteht.

## Revendications

1. Une préparation primaire pour administration intramusculaire comprenant un acide nucléique encodant un antigène: et une préparation secondaire pour administration intranasale comprenant l'antigène encapsulé dans des liposomes, permettant d'éliciter une réponse immunitaire chez un animal dans quoi l'administration de la préparation secondaire produit une réponse immunitaire comprenant:

(1) une augmentation du niveau de sérum de l'IgA spécifique de l'antigène par rapport au niveau de sérum de l'IgA spécifique de l'antigène avant l'administration de la préparation secondaire et
(2) une augmentation par rapport à un animal non traité de la proportion de lymphocytes T cytotoxiques +IFNγ+CD8 spécifiques de l'antigène dans le poumon comme dans la rate.

2. La composition selon la revendication 1 dans quoi la réponse immunitaire élicitée tend vers une réponse Th1 chez un animal hôte.

3. La composition selon la revendication 1 ou la revendication 2 dans quoi la réponse immunitaire désirée est contre un pathogène et l'antigène est un antigène spécifique du pathogène.

4. La composition selon la revendication 1 dans quoi les liposomes dans la préparation secondaire ont une taille moyenne d'environ 0,5-5 μm.

5. La composition selon la revendication 3 dans quoi la réponse immunitaire confère une immunité contre le pathogène.

6. La composition selon la revendication 5 dans quoi la réponse immunitaire protège les animaux d'une épreuve par ledit pathogène.

7. La composition selon l'une des revendications 3-6 dans quoi le pathogène est un virus.

8. La composition selon la revendication 7 dans quoi le virus est le HBV, et l'antigène spécifique du pathogène est le HbsAg.

9. La composition selon la revendication 8 dans quoi l'animal est humain.

10. La composition selon la revendication 1, dans quoi la préparation secondaire ne contient pas d'adjuvant autre qu'un liposome.

11. Un kit permettant d'éliciter une réponse immunitaire chez un animal hôte comprenant:

a) un premier composant immunisant pour administration intramusculaire comprenant un acide nucléique encodant un antigène;
b) un deuxième composant immunisant pour administration intranasale comprenant l'antigène encapsulé dans des liposomes;
c) une instruction permettant à un utilisateur d'administrer à l'animal le premier composant immunisant suivi par l'administration du deuxième composant immunisant pour éliciter une réponse immunitaire chez l'animal.

12. Le kit selon la revendication 11 dans quoi la réponse immunitaire désirée est contre un pathogène et l'antigène est un antigène spécifique du pathogène.

13. Le kit selon la revendication 11 dans quoi les liposomes dans le deuxième composant immunisant ont une taille moyenne d'environ 0,5-5 γm.

14. Le kit selon la revendication 12 ou la revendication 13 dans quoi le pathogène est un virus.

15. Le kit selon la revendication 14 dans quoi le virus est le HBV, et l'antigène spécifique du pathogène est le HBsAg.

16. Le kit selon l'une des revendications 11-15 dans quoi l'animal est humain.

17. Le kit selon la revendication 11, dans quoi le deuxième composant immunisant ne contient pas d'adjuvant autre qu'un liposome.

18. La composition selon la revendication 1, dans quoi la préparation secondaire se compose d'antigène encapsulé dans des liposomes et d'un solvant approprié.

19. Le kit selon la revendication 11, dans quoi le deuxième composant immunisant se compose d'antigène encapsulé dans des liposomes et d'un solvant approprié.

Figure 1.

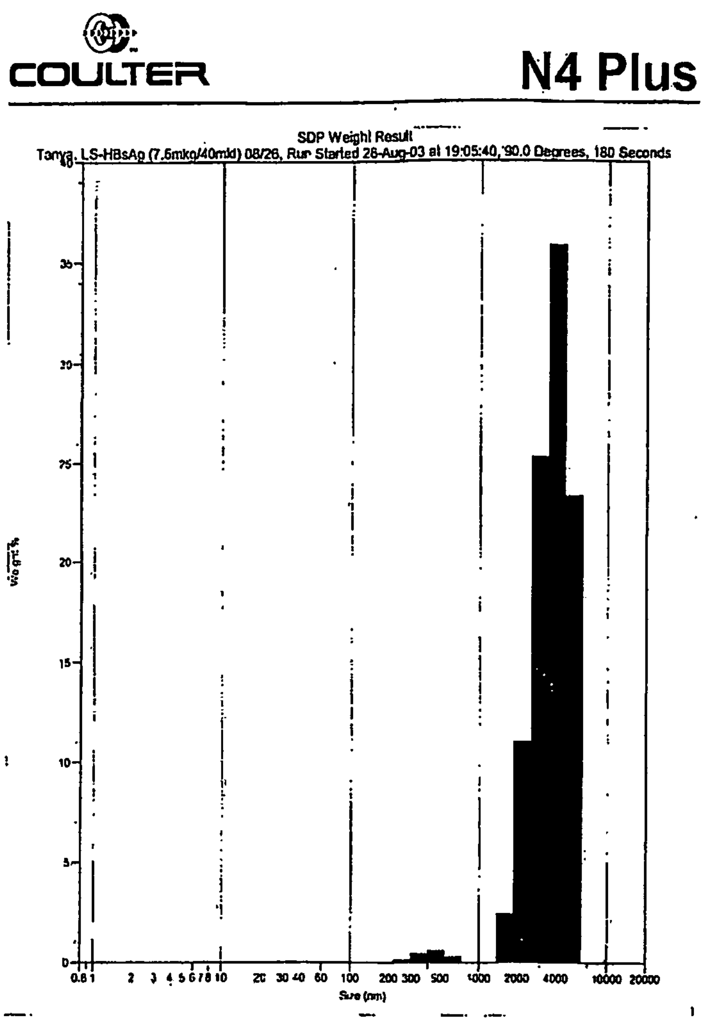
COULTER
N4 Plus
SDP Weight Result
Tanya. LS-HBsAg (7.6mkg/40mld) 08/26, Run Started 28-Aug-03 at 19:05:40, 90.0 Degrees, 180 Seconds
Weight %
0
5
10
15
20
25
30
35
40
0.8 1 2 3 4 5 6 7 8 10 20 30 40 60 100 200 300 500 1000 2000 4000 10000 20000
Size (nm)

Figure 2.

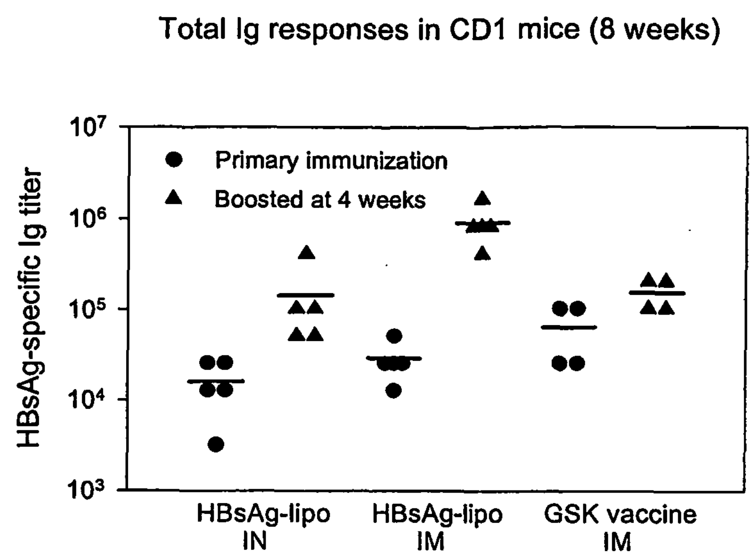
Total Ig responses in CD1 mice (8 weeks)
HBsAg-specific Ig titer
$10^7$
$10^6$
$10^5$
$10^4$
$10^3$
Primary immunization
Boosted at 4 weeks
HBsAg-lipo IN
HBsAg-lipo IM
GSK vaccine IM

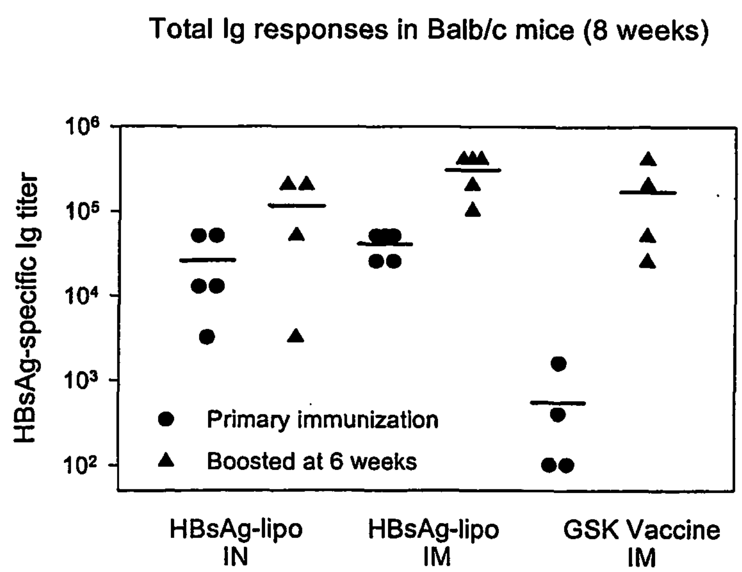
Total Ig responses in Balb/c mice (8 weeks)
HBsAg-specific Ig titer
$10^6$
$10^5$
$10^4$
$10^3$
$10^2$
Primary immunization
Boosted at 6 weeks
HBsAg-lipo IN
HBsAg-lipo IM
GSK Vaccine IM

Figure 3.

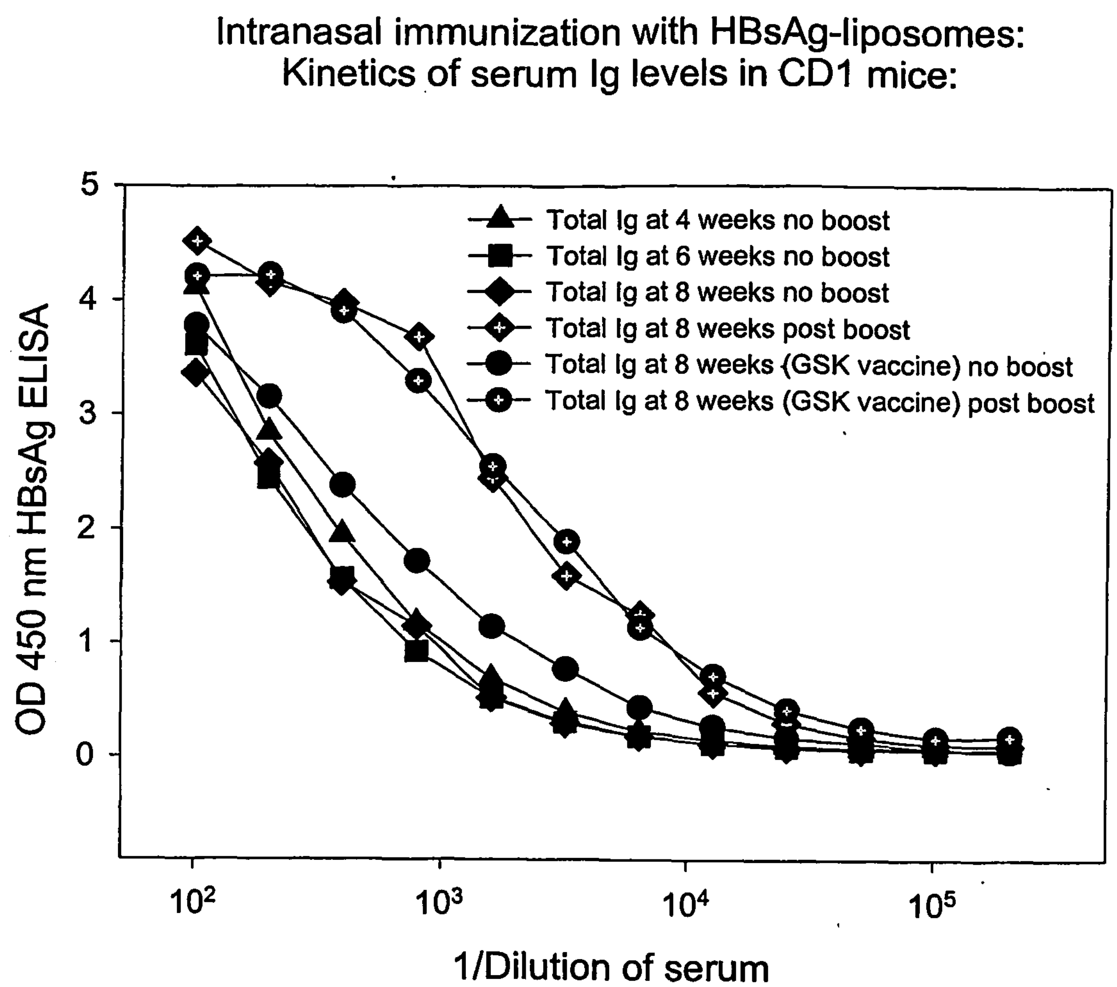
Intranasal immunization with HBsAg-liposomes:
Kinetics of serum Ig levels in CD1 mice:
Total Ig at 4 weeks no boost
Total Ig at 6 weeks no boost
Total Ig at 8 weeks no boost
Total Ig at 8 weeks post boost
Total Ig at 8 weeks (GSK vaccine) no boost
Total Ig at 8 weeks (GSK vaccine) post boost
OD 450 nm HBsAg ELISA
0
1
2
3
4
5
$10^2$
$10^3$
$10^4$
$10^5$
1/Dilution of serum

Figure 4.

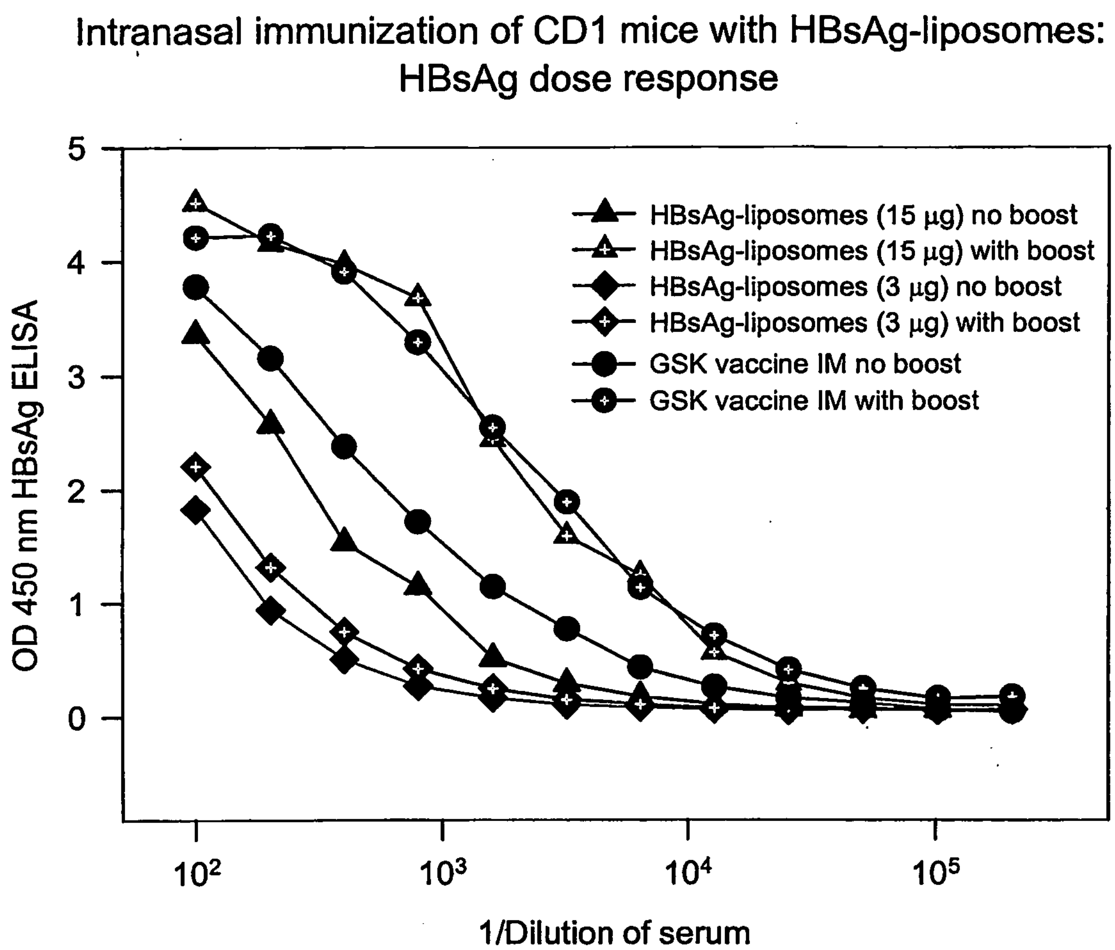
Intranasal immunization of CD1 mice with HBsAg-liposomes:
HBsAg dose response
OD 450 nm HBsAg ELISA
5
4
3
2
1
0
$10^2$
$10^3$
$10^4$
$10^5$
1/Dilution of serum
HBsAg-liposomes (15 μg) no boost
HBsAg-liposomes (15 μg) with boost
HBsAg-liposomes (3 μg) no boost
HBsAg-liposomes (3 μg) with boost
GSK vaccine IM no boost
GSK vaccine IM with boost

Figure 5.

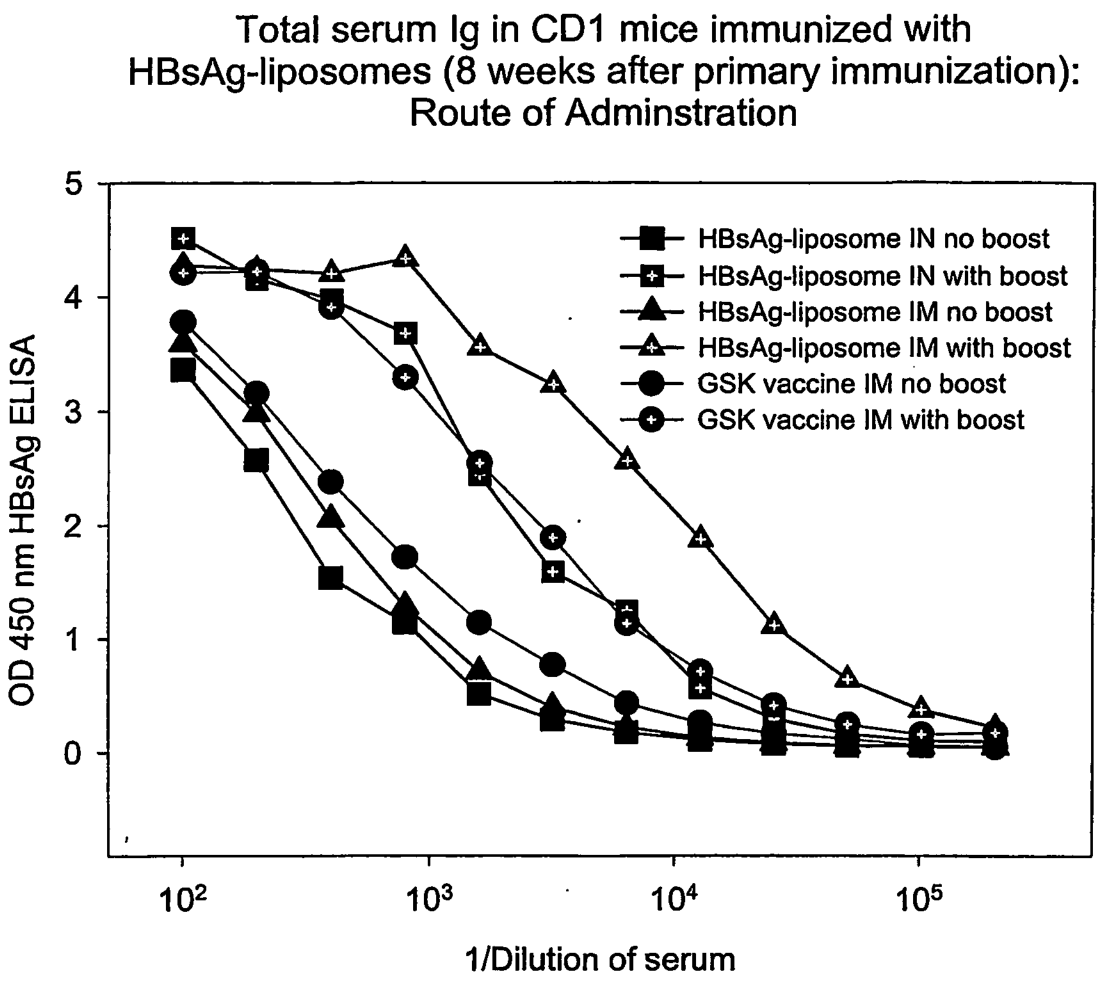
Total serum Ig in CD1 mice immunized with
HBsAg-liposomes (8 weeks after primary immunization):
Route of Adminstration
OD 450 nm HBsAg ELISA
5
4
3
2
1
0
HBsAg-liposome IN no boost
HBsAg-liposome IN with boost
HBsAg-liposome IM no boost
HBsAg-liposome IM with boost
GSK vaccine IM no boost
GSK vaccine IM with boost
$10^2$
$10^3$
$10^4$
$10^5$
1/Dilution of serum

Figure 6.

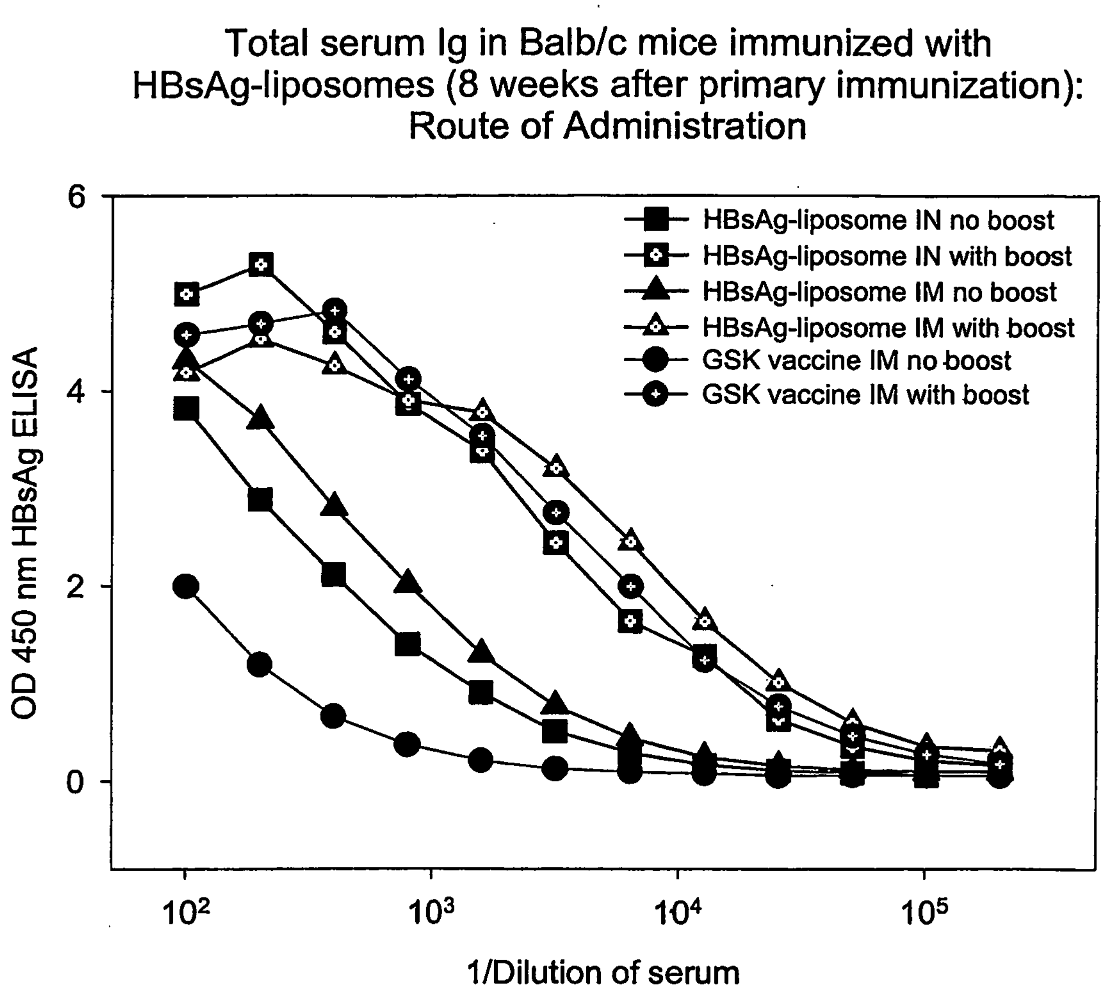
Total serum Ig in Balb/c mice immunized with
HBsAg-liposomes (8 weeks after primary immunization):
Route of Administration
OD 450 nm HBsAg ELISA
6
4
2
0
HBsAg-liposome IN no boost
HBsAg-liposome IN with boost
HBsAg-liposome IM no boost
HBsAg-liposome IM with boost
GSK vaccine IM no boost
GSK vaccine IM with boost
$10^2$
$10^3$
$10^4$
$10^5$
1/Dilution of serum

Figure 7.

Total serum Ig in CD1 mice immunized intranasally with HBsAg-liposomes: fresh versus lyophilized (8 weeks after primary immunization)

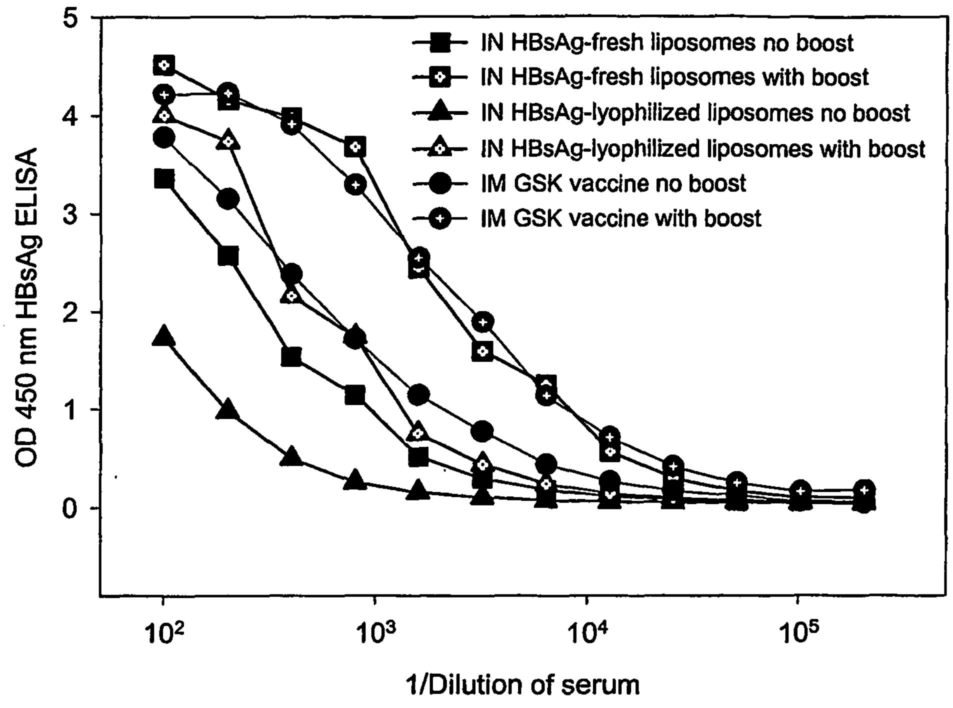

Figure 8.

Total serum Ig in CD1 mice immunized intramuscularly with HBsAg-liposomes: fresh versus lyophilized (8 weeks after primary immunization)

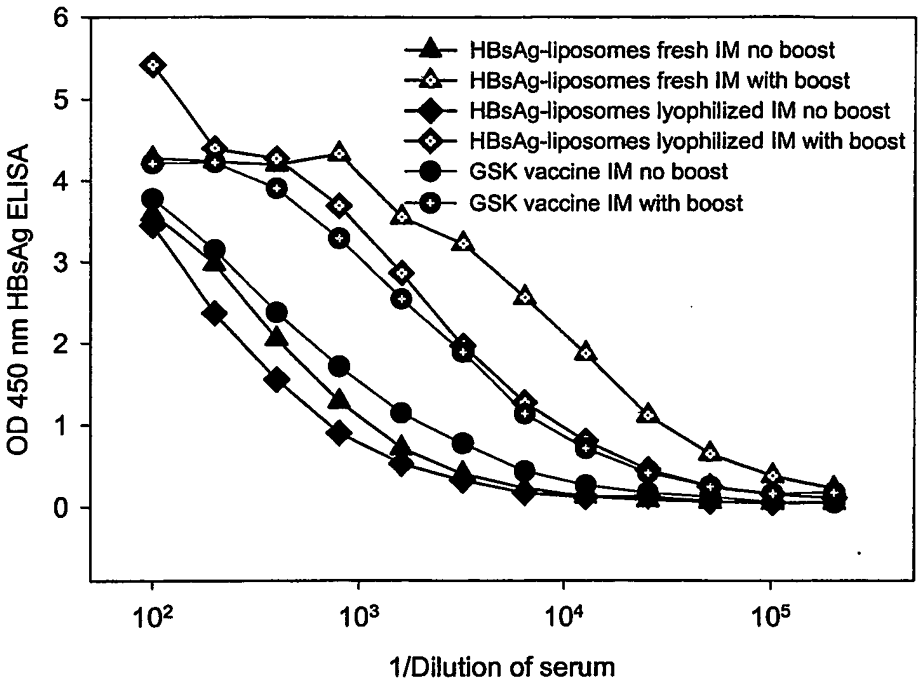

Figure 9.

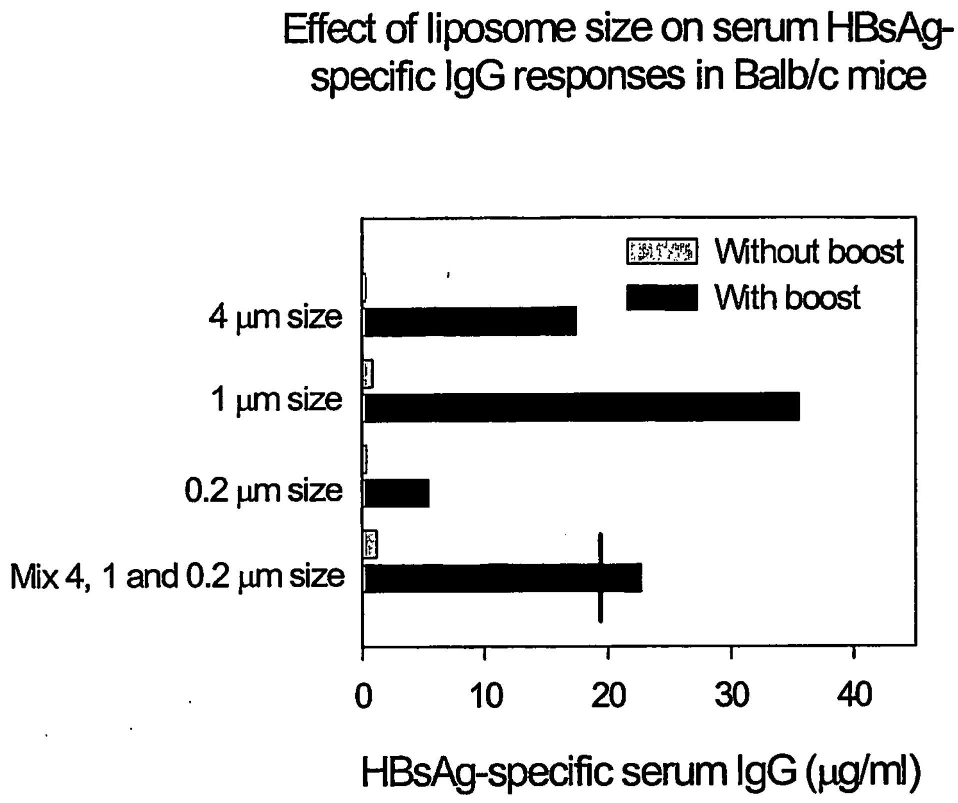
Effect of liposome size on serum HBsAg-specific IgG responses in Balb/c mice
Without boost
With boost
4 μm size
1 μm size
0.2 μm size
Mix 4, 1 and 0.2 μm size
0
10
20
30
40
HBsAg-specific serum IgG (μg/ml)

Figure 10.

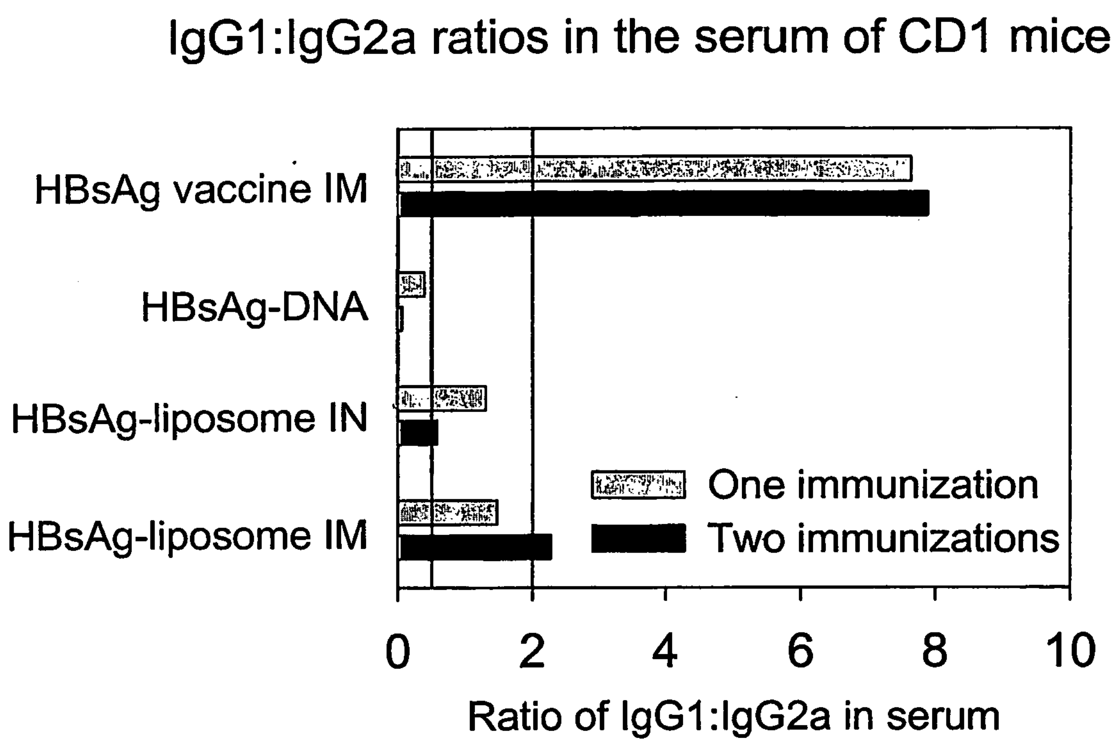
IgG1:IgG2a ratios in the serum of CD1 mice
HBsAg vaccine IM
HBsAg-DNA
HBsAg-liposome IN
HBsAg-liposome IM
One immunization
Two immunizations
0
2
4
6
8
10
Ratio of IgG1:IgG2a in serum

Figure 11.

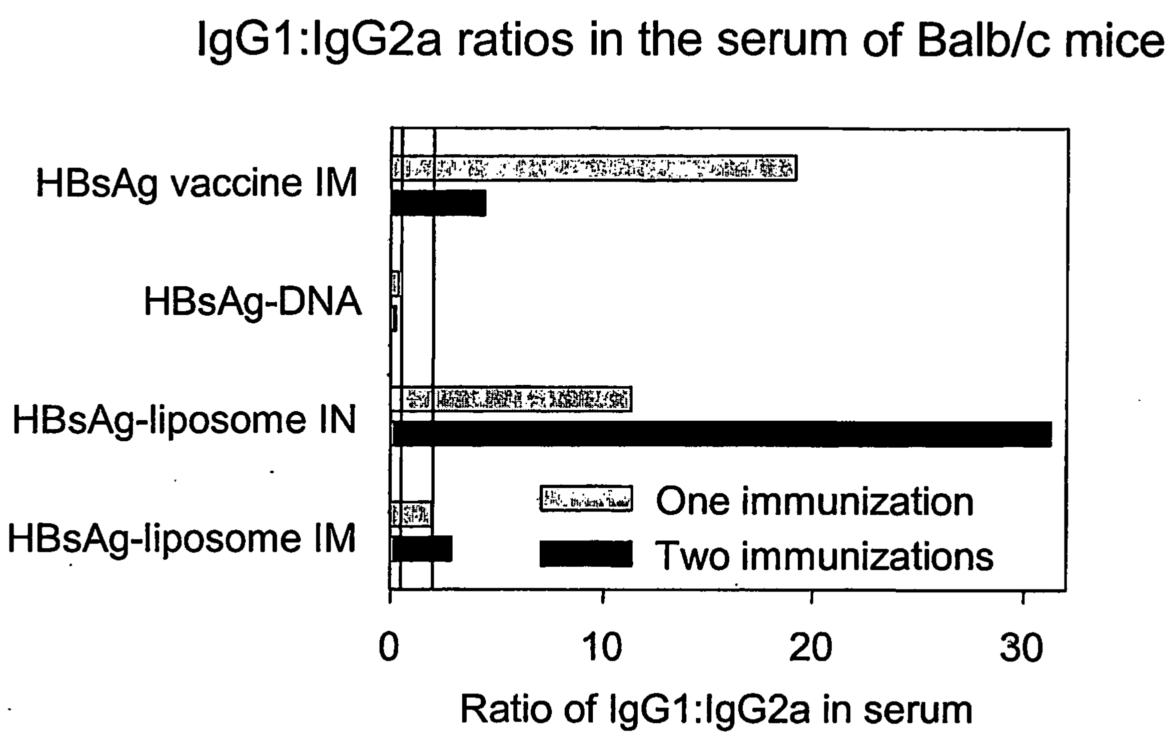
IgG1:IgG2a ratios in the serum of Balb/c mice
HBsAg vaccine IM
HBsAg-DNA
HBsAg-liposome IN
HBsAg-liposome IM
One immunization
Two immunizations
0
10
20
30
Ratio of IgG1:IgG2a in serum

Figure 12.

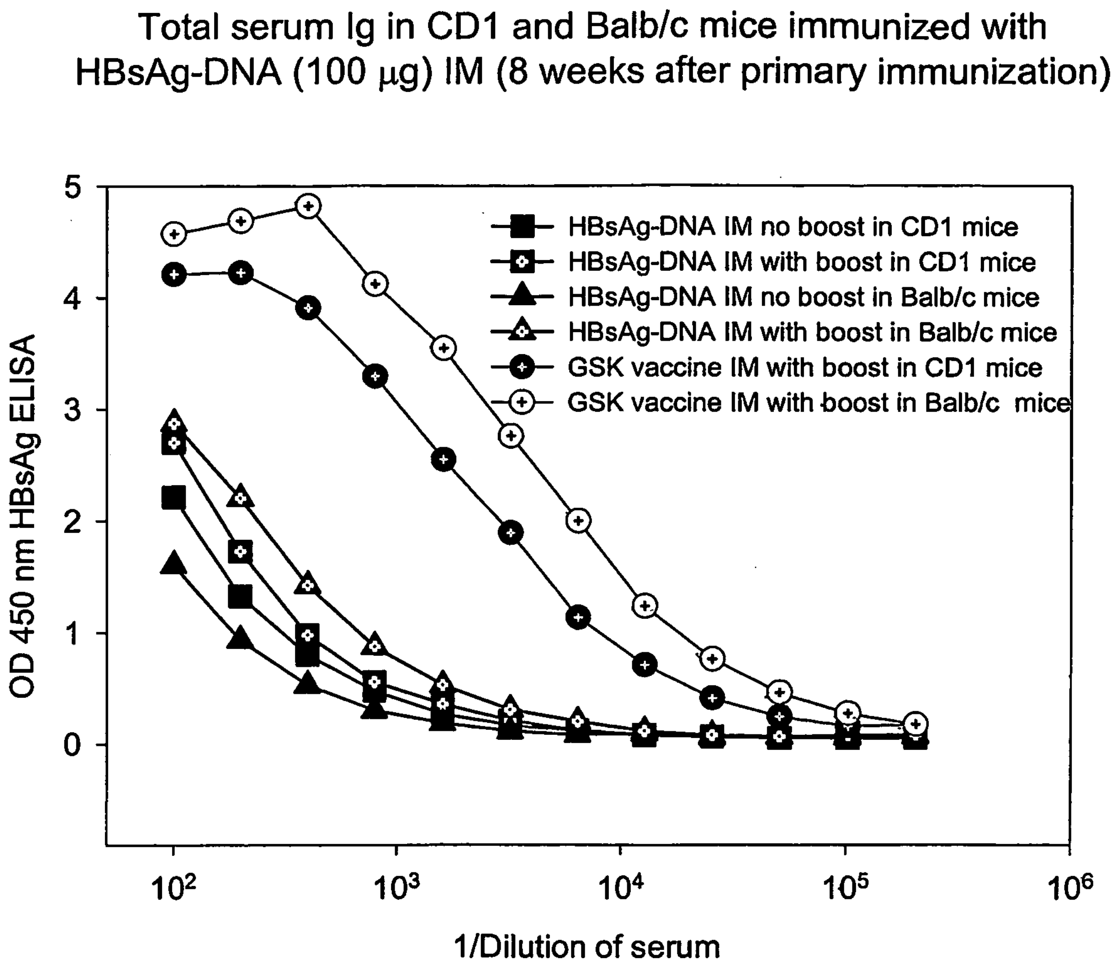
Total serum Ig in CD1 and Balb/c mice immunized with
HBsAg-DNA (100 µg) IM (8 weeks after primary immunization)
OD 450 nm HBsAg ELISA
5
4
3
2
1
0
$10^2$
$10^3$
$10^4$
$10^5$
$10^6$
1/Dilution of serum
HBsAg-DNA IM no boost in CD1 mice
HBsAg-DNA IM with boost in CD1 mice
HBsAg-DNA IM no boost in Balb/c mice
HBsAg-DNA IM with boost in Balb/c mice
GSK vaccine IM with boost in CD1 mice
GSK vaccine IM with boost in Balb/c mice

Figure 13.

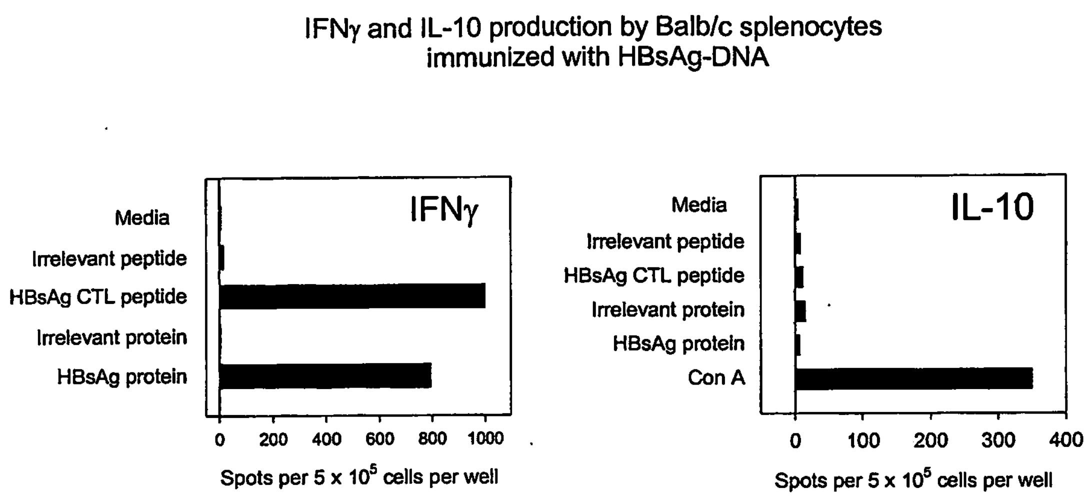
IFNγ and IL-10 production by Balb/c splenocytes immunized with HBsAg-DNA
IFNγ
Media
Irrelevant peptide
HBsAg CTL peptide
Irrelevant protein
HBsAg protein
0
200
400
600
800
1000
Spots per 5 x 10^5 cells per well
IL-10
Media
Irrelevant peptide
HBsAg CTL peptide
Irrelevant protein
HBsAg protein
Con A
0
100
200
300
400
Spots per 5 x 10^5 cells per well

Figure 14.

Heterologous immunization induces high levels of HBsAg-specific serum Ig

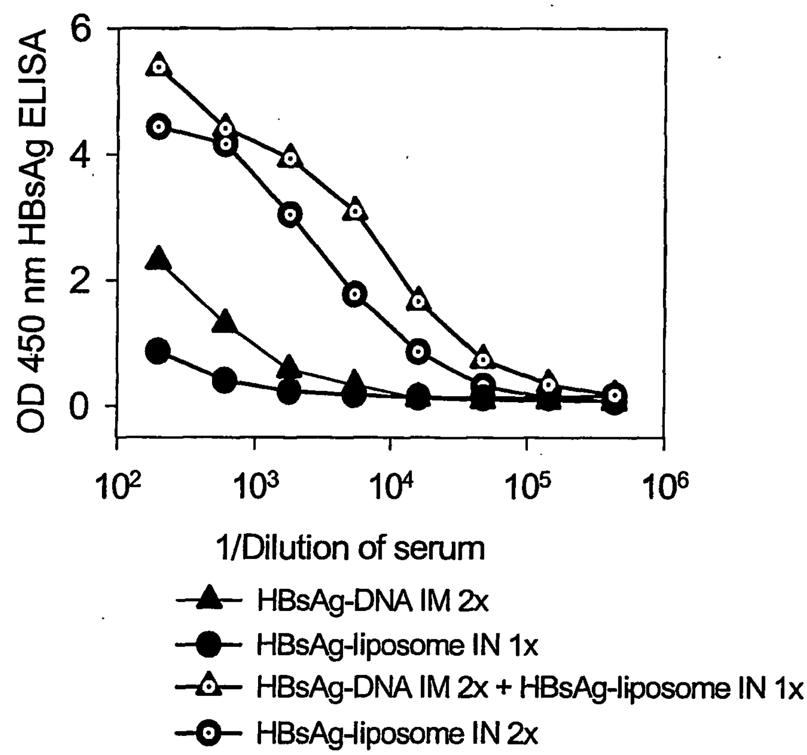

Figure 15.

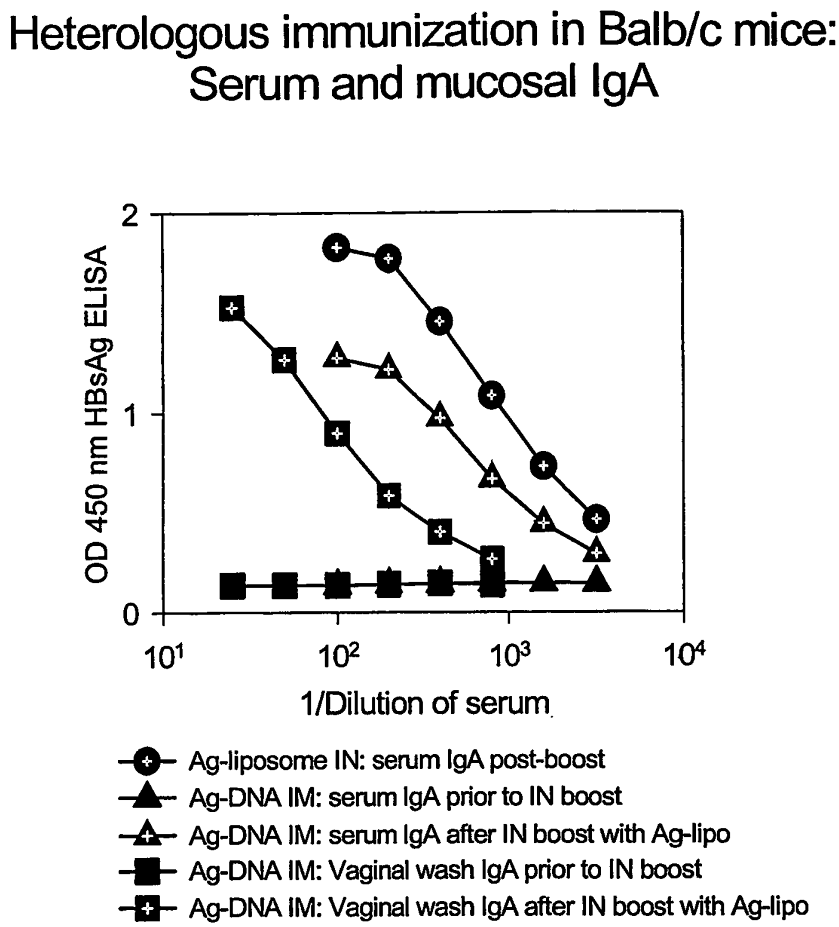
Heterologous immunization in Balb/c mice:
Serum and mucosal IgA
OD 450 nm HBsAg ELISA
2
1
0
$10^1$
$10^2$
$10^3$
$10^4$
1/Dilution of serum
Ag-liposome IN: serum IgA post-boost
Ag-DNA IM: serum IgA prior to IN boost
Ag-DNA IM: serum IgA after IN boost with Ag-lipo
Ag-DNA IM: Vaginal wash IgA prior to IN boost
Ag-DNA IM: Vaginal wash IgA after IN boost with Ag-lipo

Figure 16.

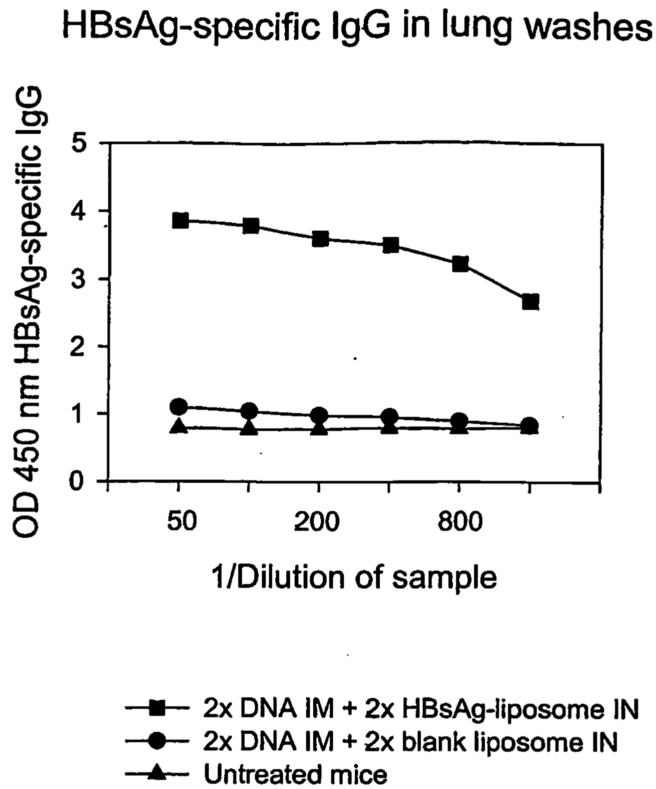
HBsAg-specific IgG in lung washes
OD 450 nm HBsAg-specific IgG
5
4
3
2
1
0
50
200
800
1/Dilution of sample
2x DNA IM + 2x HBsAg-liposome IN
2x DNA IM + 2x blank liposome IN
Untreated mice

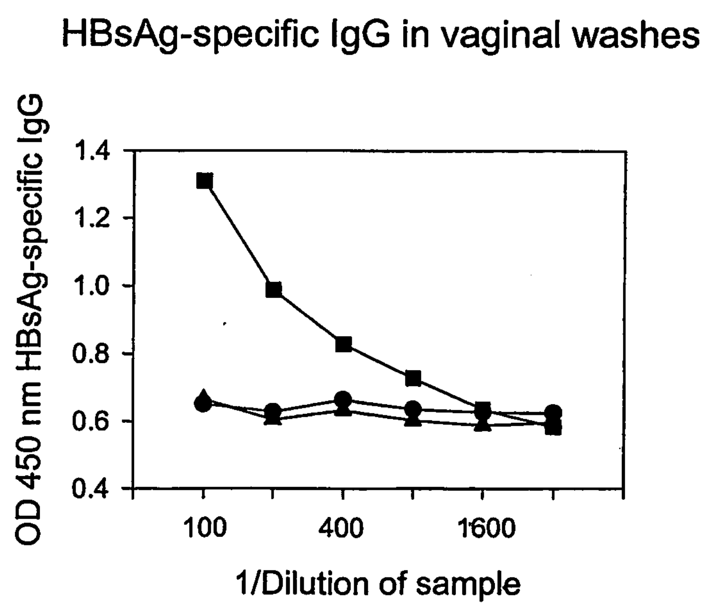
HBsAg-specific IgG in vaginal washes
OD 450 nm HBsAg-specific IgG
1.4
1.2
1.0
0.8
0.6
0.4
100
400
1600
1/Dilution of sample

Figure 17.

Th1 biased responses in individual mice
after heterologous immunization

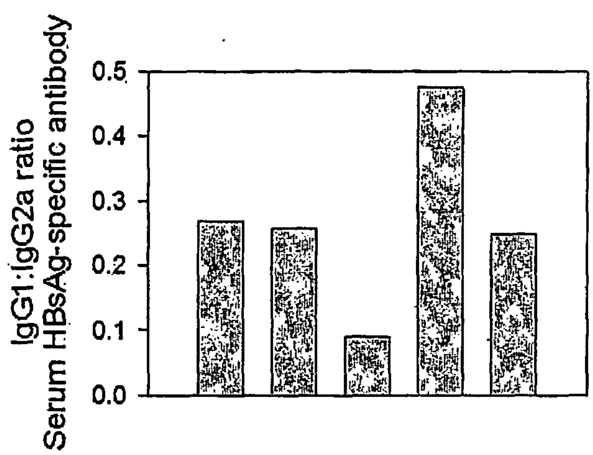


IgG1:IgG2a ratios ≤ 0.5 define a type 1 helper T cell phenotype.
Data are from individual mice.

Figure 18.

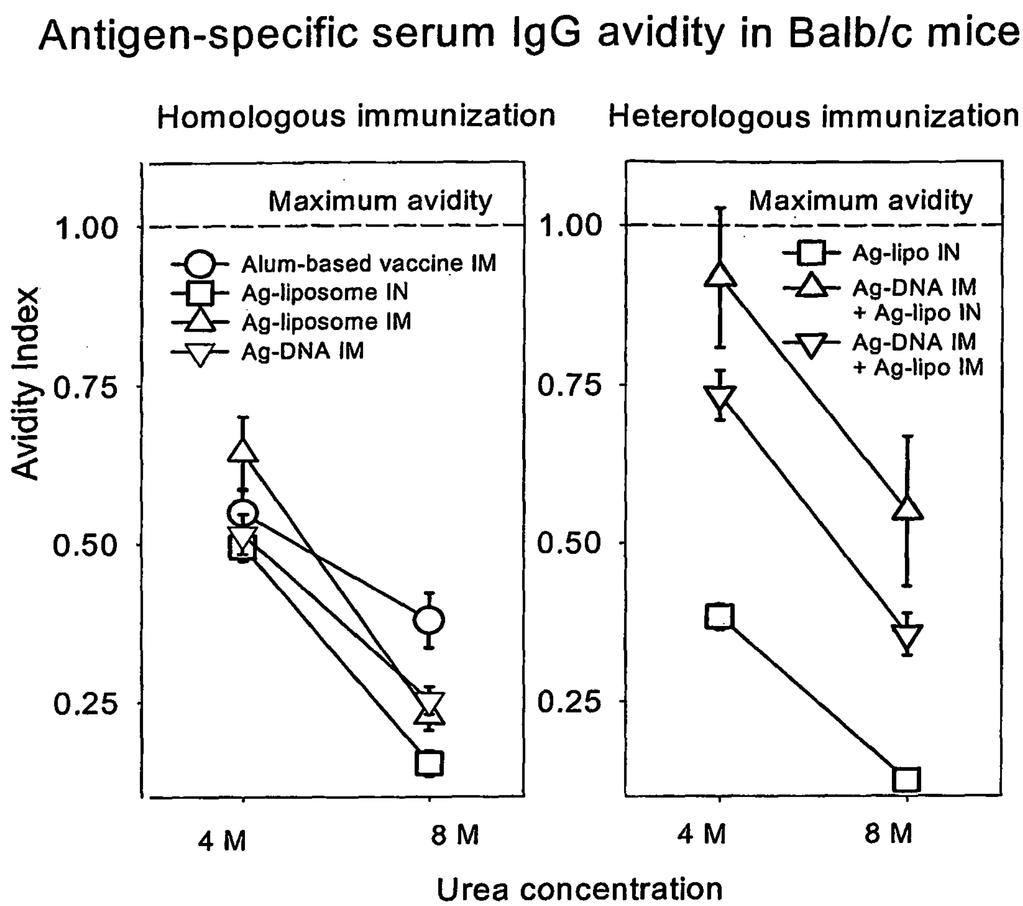
Antigen-specific serum IgG avidity in Balb/c mice
Homologous immunization
Heterologous immunization
Maximum avidity
Maximum avidity
1.00
0.75
0.50
0.25
Avidity Index
Alum-based vaccine IM
Ag-liposome IN
Ag-liposome IM
Ag-DNA IM
Ag-lipo IN
Ag-DNA IM + Ag-lipo IN
Ag-DNA IM + Ag-lipo IM
4 M
8 M
Urea concentration

Figure 19.

Prime-boost regimen induces potent CTL activity

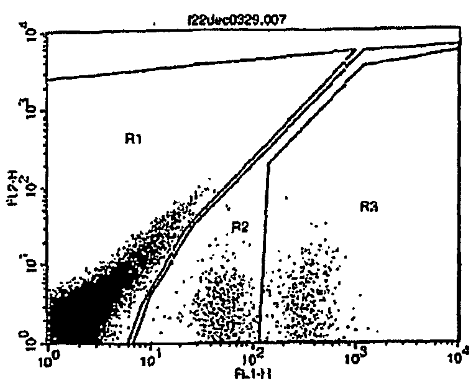


Naïve Balb/c mice

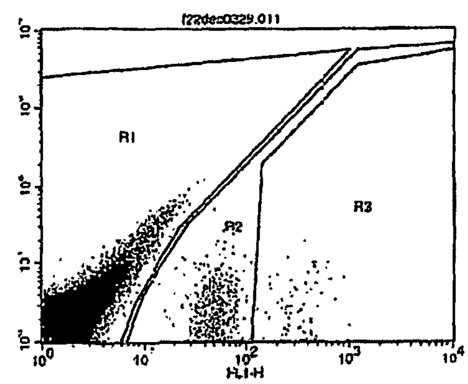


Balb/c mice immunized with HBsAg-DNA IM followed by HBsAg-liposomes IN

R2: CFSE$^{low}$ unpulsed donor population
R3: CFSE$^{high}$ HBsAg peptide pulsed donor population

Figure 20.

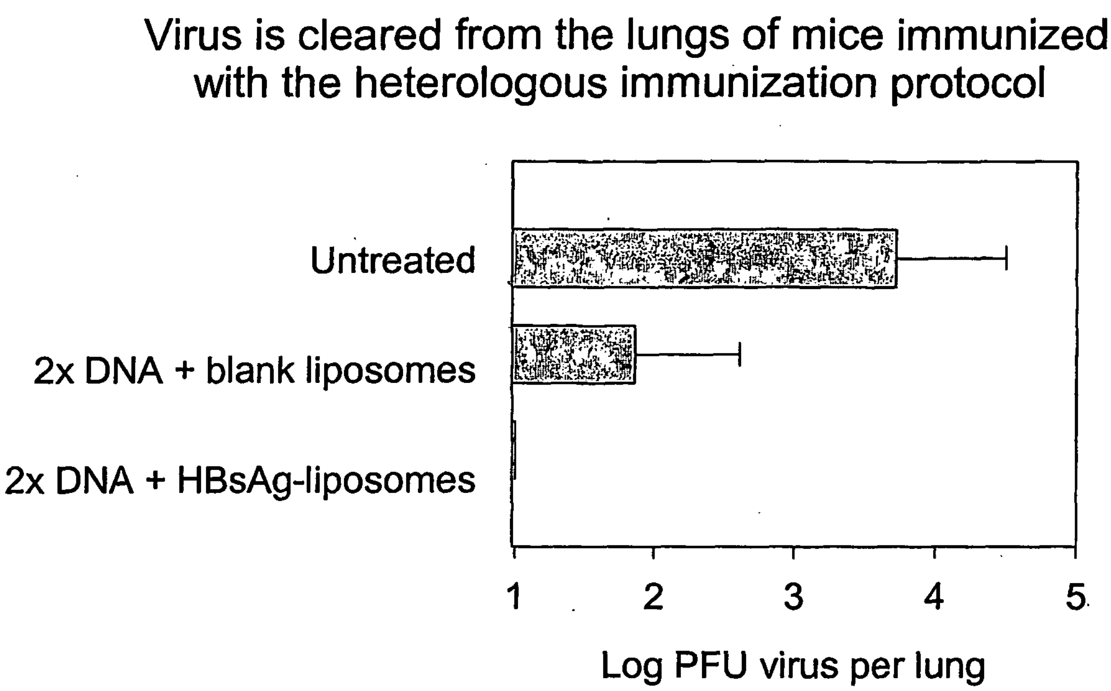
Virus is cleared from the lungs of mice immunized with the heterologous immunization protocol
Untreated
2x DNA + blank liposomes
2x DNA + HBsAg-liposomes
1
2
3
4
5
Log PFU virus per lung

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 53492304 P
- WO 0011140 A
- WO 9951748 A
- WO 9917741 A
- EP 366412 A
- WO 9928475 A
- WO 9006951 A
- WO 9903884 A
- US 5843464 A
- EP 9202591 W
- WO 9310152 A
- GB 9124390 A
- GB 8900895 W
- WO 9001496 A
- WO 9927944 A
- US 4837028 A
- WO 8804924 A, Allen
- US 5543152 A, Webb
- WO 9713499 A, Lim
- US 4426330 A
- US 4534899 A
- EP 0445131 B1
- WO 9004384 A, Fisher
- US 5013556 A, Woodle
- US 5356633 A
- US 5213804 A, Martin
- EP 0496813 B1
- WO 9105545 A
- US 5225212 A, Martin
- WO 9420073 A, Zalipsky
- WO 9610391 A, Choi
- US 5540935 A, Miyazaki
- US 5556948 A, Tagawa
- US 6632663 B
- WO 03075955 A1
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A


### Non-patent literature cited in the description


- **Csencsits KL et al.** *Jl.,* 1999, 1382-1389
- **Liu M et al.** *J Immunology,* 1982, vol. 129 (6), 2653-2661
- **Bice DE et al.** *Int Arch Allergy Appl Immunol.,* 1980, vol. 63 (4), 438-45
- **Bice DE et al.** *Am J Respir Cell Mol Biol.,* 1993, vol. 8 (6), 662-667
- **Thompson AH.** *Vaccine,* vol. 17, 1404-15
- **Liu et al.** *Ann. N.Y. Acad. Sci.,* 1995, 772
- **Pardoll ; Beckerieg.** *Immunity,* 1995, vol. 3, 165
- **McDonnell ; Askari.** *N. Engl. J. Med.,* 1996, vol. 334, 42
- **Manickan et al.** *J. Immunol.,* 1995, vol. 155, 259
- **Sedegah et al.** *Proc. Natl. Acad. Sci. USA,* 1994, vol. 91, 9866
- **Barry et al.** *Nature,* 1995, vol. 377, 632
- **Xu ; Liew.** *Vaccine,* 1994, vol. 12, 1534
- *Biochem Biophys Acta,* 1989, vol. 67, 1007
- **Rubins et al.** *Microbial Pathogenesis,* vol. 25, 337-342
- **Maniatis et al.** Molecular Cloning: A Laboratory Manual. Cold Spring Harbour Press, 1989, vol. 1-3
- **Haynes et al.** *J. Biotechnology,* 1996, vol. 44, 37-42
- **Im ; Hanke.** *Expert, Rev. Vaccines,* 2004, vol. 3, S89-S97
- **Basak et al.** *Viral Immunol.,* 2004, vol. 17, 182-196
- **Doria-Rose ; Haigwood.** *Methods,* 2003, vol. 31, 207-216

- **Leifert et al.** *Immunol. Rev.,* 2004, vol. 199, 40-53
- **Alving.** *Vaccine,* 2002, vol. 20 (3), 556-S64
- **Hunter.** *Vaccine,* 2002, vol. 20 (3), 7-12
- **Alving.** *Vaccine,* 2002, vol. 20 (3), S56-S64
- **Petrovsky ; Aguilar.** *Immunol. Cell Biol.,* 2004, vol. 82, 488-496
- **Berzofsky et al.** *J. Clin. Invest.,* 2004, vol. 114, 450-462
- **Belardelli et al.** *Cancer Res.,* 2004, vol. 64, 6827-6830
- **Allen et al.** *FEBS Letters,* 1987, vol. 223, 42
- **Wu et al.** *Cancer Research,* 1993, vol. 53, 3765
- **Papahadjopoulos et al.** *Ann. N.Y. Acad. Sci.,* 1987, vol. 507, 64
- **Gabizon et al.** *Proc. Natl. Acad. Sci. USA,* 1988, vol. 85, 6949
- **Sunamoto et al.** *Bull. Chem. Soc. Jpn.,* 1980, vol. 53, 2778
- **Illum et al.** *FEBS Letters,* 1984, vol. 167, 79
- **Klibanov et al.** *FEBS Letts.,* 1990, vol. 268, 235
- **Blume et al.** *Biochimica et Biophysica Acta,* 1990, vol. 1029, 91
- **Akhtar et al.** *Nucl. Acids Res.,* 1991, vol. 19, 5551
- **Yachi et al.** *Biopharm. Drug Dispos.,* 1996, vol. 17, 699
- **Farmer et al.** *Meth. Enz.,* 1987, vol. 149, 184
- **Brodt et al.** *Cancer Immunol. Immunother.,* 1989, vol. 28, 54
- **Perez-Soler et al.** *J. Nuclear Med.,* 1985, vol. 26, 743
- **Wasan et al.** *Antimicrobial Agents and Chemotherapy,* 1993, vol. 37, 246
- **Li et al.** *Oncology Res.,* 1995, vol. 7, 611
- **Estcourt, M. J. et al.** *Int. Immunol.,* 2002, vol. 14 (1), 31-37
- **Barber, D. L. et al.** *J. Immunol.,* 2003, vol. 171, 27-31
- **Kumaraguru, U. et al.** *J. Immunol.,* 2004, vol. 172, 3719-3724
- **Smith ; Mackett ; Moss.** *Nature,* 1983, vol. 302, 490-495
- **Pancholi, P. et al.** *J. Virol.,* 2004, vol. 77 (1), 382-90
- **Marata, K. et al.** *Proc. Natl. Acad. Sci. USA,* 2003, vol. 100 (11
- **Belyakov, IM et al.** *Proc. Natl. Acad. Sci USA,* 1998, vol. 95 (4), 1709-14
- **Taylor-Robinson.** *Int J Parasitol.,* 1998, vol. 28 (1), 135-48
- **Davis, H.L. ; M. L. Michel ; R. G. Whalen.** DNA-based immunization for Hepatitis B induces continuous secretion of antigen and high levels of circulating antibody. *Human Molecular Genetics,* 1993, vol. 2, 1847-1851
- **Ishikawa, T. et al.** *J. Immunol.,* 1998, vol. 161, 5842
- **Parr, E.L. et al.** *J. Virology,* 1997, vol. 71, 8109-8115
- **Tamura, S. ; T. Kurata.** *Jpn. J. Infect. Dis.,* 2004, vol. 57 (6), 236-47
- **Renegar, K.B. et al.** *J. Inmunol.,* 2004, vol. 173 (3), 1978-86
- **Schirmbeck, R. et al.** *J. Virol.,* vol. 69 (10), 5929-34
- **Sambrook et al.** Molecular Cloning: A laboratory Manual. 1989
- Current Protocols in Molecular Biology. 1994, vol. I-III
- **Ausubel et al.** Current Protocols in Molecular Biology. John Wiley and Sons, 1989
- **Perbal.** A Practical Guide to Molecular Cloning. John Wiley & Sons, 1988
- Recombinant DNA. **Watson et al.** Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **Perbal, B.** A Practical Guide to Molecular Cloning. 1984
- Methods in Enzymology. Academic Press, vol. 1-317
- PCR Protocols: A Guide To Methods And Applications. Academic Press, 1990
- **Marshak et al.** Strategies for Protein Purification and Characterization-A Laboratory Course Manual. CSHL Press, 1996